(19) Europäisches Patentamt
European Patent Office
Office européen des brevets

(11) EP 1 250 331 B1

(12) **EUROPÄISCHE PATENTSCHRIFT**

(45) Veröffentlichungstag und Bekanntmachung des Hinweises auf die Patenterteilung:
**03.11.2004 Patentblatt 2004/45**

(21) Anmeldenummer: **01902346.4**

(22) Anmeldetag: **22.01.2001**

(51) Int Cl.[7]: **C07D 307/83**, A61K 7/48, A61K 31/343, A23L 3/3544, A61P 39/06

(86) Internationale Anmeldenummer:
**PCT/EP2001/000652**

(87) Internationale Veröffentlichungsnummer:
**WO 2001/055128 (02.08.2001 Gazette 2001/31)**

(54) **BENZOFURANONDERIVATE ENTHALTENDE FORMULIERUNG ZUM SCHUTZ VOR OXIDATIVEM STRESS**

FORMULATION FOR PROTECTION AGAINST OXIDATIVE STRESS CONTAINING BENZOFURANONE DERIVATIVES

FORMULATION DE PROTECTION CONTRE LES AGRESSIONS OXYDANTES, CONTENANT DES DERIVES DE BENZOFURANONE

(84) Benannte Vertragsstaaten:
**AT BE CH CY DE DK ES FI FR GB GR IE IT LI LU MC NL PT SE TR**

(30) Priorität: **28.01.2000 DE 10003785**
**11.10.2000 DE 10050237**

(43) Veröffentlichungstag der Anmeldung:
**23.10.2002 Patentblatt 2002/43**

(73) Patentinhaber: **MERCK PATENT GmbH**
**64293 Darmstadt (DE)**

(72) Erfinder:
- **PFLÜCKER, Frank**
**64825 Darmstadt (DE)**
- **BUCHHOLZ, Herwig**
**60599 Frankfurt (DE)**
- **ROSSKOPF, Ralf**
**64839 Münster (DE)**
- **BÜNGER, Joachim**
**64823 Gross-Umstadt (DE)**

(56) Entgegenhaltungen:
**EP-A- 0 113 534**
**CH-A- 644 603**
**DE-A- 4 342 560**

EP 1 250 331 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft kosmetische oder pharmazeutische Formulierungen sowie Nahrungsmittel und Nahrungsmittelergänzungsstoffe, die einen verbesserten Schutz gegen Oxidation aufweisen. Weiter betrifft die Erfindung neue Verbindungen mit antioxidativen Eigenschaften sowie ihre Verwendung als Antioxidationsmittel.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verhindern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also nur in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfaßt UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachläßt.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Substanzen, welche Abwehr- und Reparaturfunktionen der Haut unterstützen oder ersetzen sollen, müssen an ihren Wirkungsort transportiert werden können. Dazu stehen prinzipiell zwei Wege offen.

Entweder wird die Substanz auf die Haut aufgetragen und dringt durch die äußeren Schich-ten in tiefere Schichten der Haut vor oder der Wirkstoff wird, z.B. nach oraler Aufnahme, über die Blutbahn zum Wirkort transportiert.

Eine weitere Schwierigkeit bei der Herstellung von Kosmetika besteht darin, dass Wirkstoffe, die in kosmetische Formulierungen eingearbeitet werden sollen, oftmals nicht stabil sind und in der Formulierung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

In der DE 197 10 854 A1 wird die Verwendung von Benzophenonen und deren Derivaten gegen die UV-induzierte Zersetzung von Dibenzoylmethan und dessen Derivaten beschrieben.

In der DE 197 46 654 A1 werden photostabile UV-Filter enthaltende kosmetische und pharmazeutische Zubereitungen beschrieben. Dabei werden 4,4-Diarylbutadiene als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen verwendet.

In der DE 195 08 608 A1 wird ein lichtbeständiges kosmetisches Mittel beschrieben. Es werden kosmetische Mittel zum Schutz vor UV-Strahlen der Wellenlänge zwischen 280 und 400 nm offenbart, die mindestens ein Tetraalkylquercetin, in einem kosmetisch annehmbaren Medium auf Ölbasis enthalten.

In der DE 197 55 504 A1 wird die Verwendung von Flavonen und Flavonoiden gegen die UV-induzierte Zersetzung von Dibenzoylmethan und dessen Derivaten beschrieben.

In der DE 197 50 030 A1 wird eine Butylmethoxydibenzoylmethan als Lichtschutzfilter enthaltende kosmetische Zubereitung beschrieben. Zur Verbesserung der Photostabilität sind der kosmetischen Zubereitung im UVA-Bereich absorbierende, nicht lösliche anorganische Partikel zugesetzt. Als nicht lösliche anorganische Partikel werden mikrofeine Partikel verwendet, die ausgewählt sind aus der Gruppe Titandioxid, Eisenoxid, Ceroxid und Zinkoxid.

In der US 4,532,257 werden substituierte Coumaranone beschrieben, wobei der Benzofurananteil entweder unsubstituiert ist oder eine Carboxylgruppe sowie eine Alkyl- oder Alkenylgruppen trägt. Für die Benzylgruppe werden verschiedene Substituienten beschrieben. Die Verbindungen werden als antiallergische oder antiinflammatorische Wirkstoffe vorgeschlagen, insbesondere zur Behandlung von Asthma.

In der GB 2 131 431 werden Coumaranonderivate beschrieben, wobei diese unter anderem auch durch $C_{1-4}$-Alkoxygruppen substituiert sein können. Die Verbindungen werden ebenfalls zur Behandlung allergischer oder entzündlicher Erkrankungen vorgeschlagen.

In der WO 91/17749 werden Wachstumshemmer zur Behandlung von Krebs vorgeschlagen. Unter anderem werden auch Aurone vorgeschlagen, wobei das Grundgerüst durch Hydroxy- oder Methoxygruppen substituiert sein kann.

In der FR 1.502.727 wird die Herstellung von Aryl-2-amino-3-benzofuran und von Aryl-2-benzofuranon-3(2H) beschrieben.

Aufgabe der Erfindung ist es daher, eine kosmetische oder pharmazeutische Zusammensetzung zur Verfügung zu stellen, welche eine schützende Wirkung gegen UV-Strahlen aufweist und/oder eine schützende Wirkung gegen oxidativen Stress auf Körperzellen ausübt und/oder einer Alterung der Haut entgegenwirkt.

Diese Aufgabe wird gelöst durch eine kosmetische oder pharmazeutische Formulierung, enthaltend zumindest eine Verbindung der Formel I

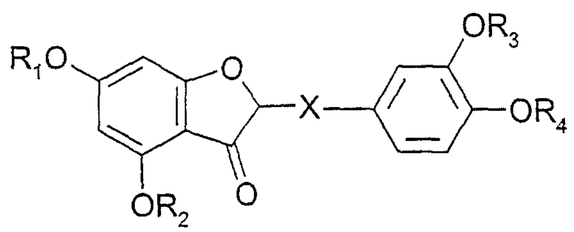

I

wobei -X- steht für eine Einfachbindung, $-CH_2-$, =CH-, -C(O)-, $=C(OR^5)-$, $-C(NR^5)-$, $-CH(NR^5R^6)-$, $-CH(OR^5)-$ und $R^1$, $R^2$, $R^3$, $R^4$, $R^5$, und $R^6$ gleich oder verschieden sein können, und unabhängig voneinander stehen für

- H
- geradkettige oder verzweigte $C_1$- bis $C_{12}$-Alkyl und/oder Alkylcarbonylgruppen,
- geradkettige oder verzweigte $C_3$- bis $C_{12}$-Alkenyl und/oder -Alkenylcarbonylgruppen,
- geradkettige oder verzweigte $C_1$- bis $C_{12}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- $C_3$- bis $C_{10}$-Cycloalkyl- und/oder Cycloalkylcarbonylgruppen und $C_3$- bis $C_{12}$-Cycloalkenyl- und/oder Cycloalkenylcarbonylgruppen, wobei die Ringe jeweils auch durch $-(CH_2)_n$-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste,

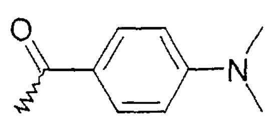

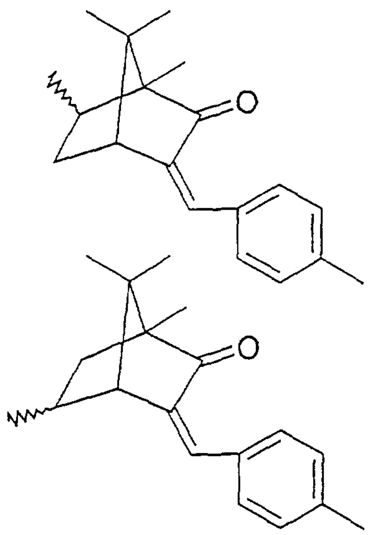

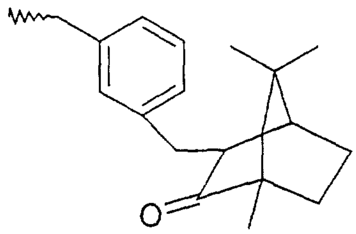

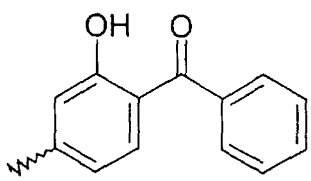

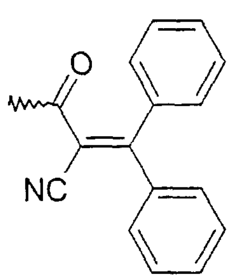

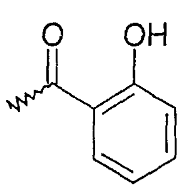

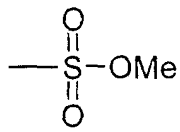

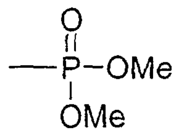

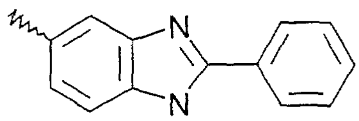

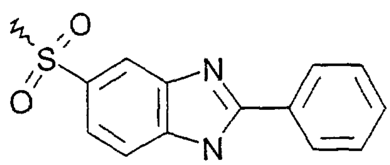

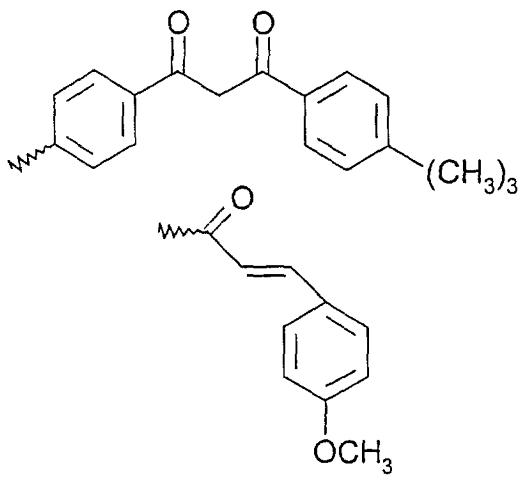

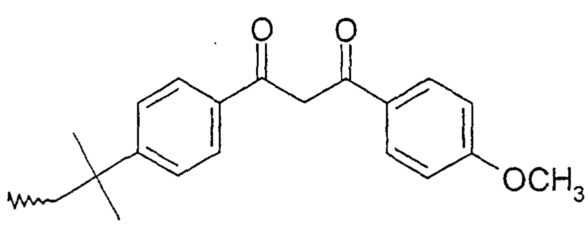

wobei Me steht für ein Proton oder ein Alkalimetallion, insbesondere ein Natrium- oder Kaliumion.

Die Reste können also als Ether oder als Ester an den Grundkörper gebunden sein.

Die positive Wirkung der erfindungsgemäßen kosmetischen oder pharmazeutischen Zubereitung beruht vermutlich auf der Wirkung der Verbindungen der Formel I als Antioxidationsmittel bzw. als Radikalfänger. Um ihre positive

Wirkung auf die Haut entwickeln zu können, sollten die Verbindungen der Formel I in tiefere Hautschichten vordringen können. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass die Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.

Aufgrund dieser Eigenschaften eignen sich die erfindungsgemäßen Formulierungen bzw. die erfindungsgemäßen Verbindungen allgemein zur Immunprotektion und zum Schutz der DNA und RNA. Insbesondere eignen sich die Formulierzungen bzw. Verbindungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Formulierungen bzw. der erfindungsgemäßen Verbindungen ist der Zellschutz, insbesondere der Schutz von Langerhans-Zellen vor Schäden durch die oben genannten Einflüsse. Alle diese Verwendungen sind ausdrücklich auch Gegenstand der vorliegenden Erfindung.

Efindungsgemäß bevorzugt sind kosmetische oder pharmazeutische Formulierungen, die Verbindungen nach Formel I enthalten, wobei -Xsteht für eine Einfachbindung, $-CH_2-$, -C(O)-, $=C(OR^5)-$, $-C(NR^5)-$, $-CH(NR^5R^6)-$ oder $-CH(OR^5)-$.

Weiterhin bevorzugt sind kosmetische oder pharmazeutische Formulierungen, die Verbindungen nach Formel I enthalten, wobei -Xsteht für =CH- und mindestens ein Rest aus $R^1$, $R^2$, $R^3$ und $R^4$ steht für eine Gruppe, die ausgewählt ist aus der folgenden Liste

- geradkettige oder verzweigte $C_1$- bis $C_{12}$-Alkylcarbonylgruppen,
- geradkettige oder verzweigte $C_3$- bis $C_{12}$-Alkenylcarbonylgruppen,
- geradkettige oder verzweigte $C_1$- bis $C_{12}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- $C_3$- bis $C_{10}$-Cycloalkylcarbonylgruppen und $C_3$- bis $C_{12}$-Cycloalkenyl- und/oder Cycloalkenylcarbonylgruppen, wobei die Ringe jeweils auch durch $-(CH_2)_n$-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste,

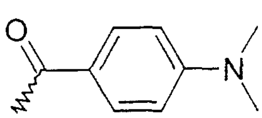

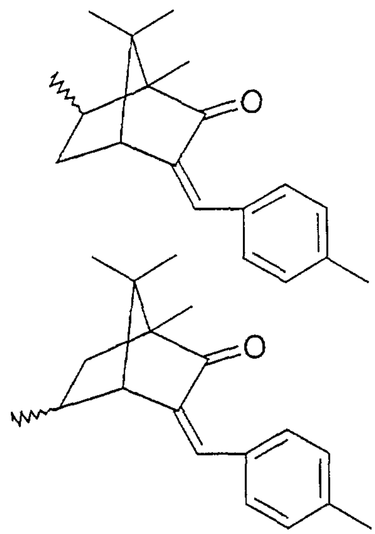

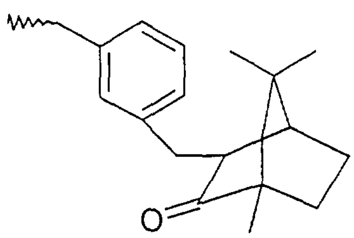

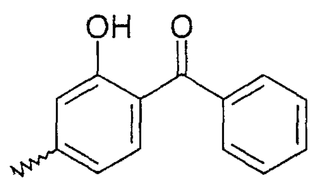

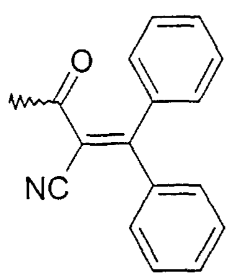

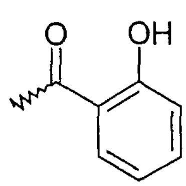

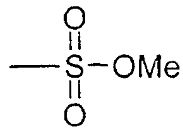

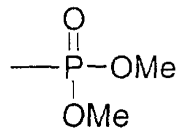

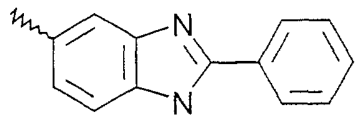

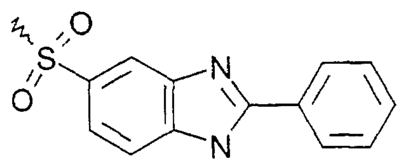

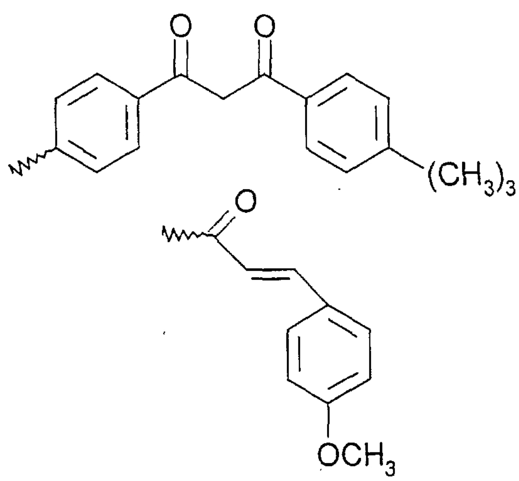

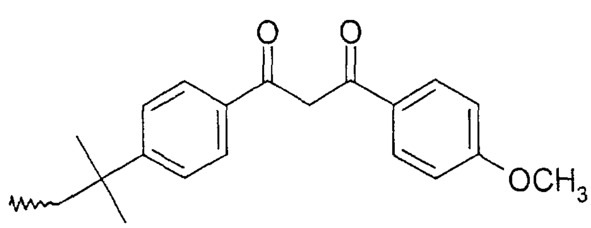

wobei Me steht für ein Proton oder ein Alkalimetallion, insbesondere ein Natrium- oder Kaliumion.

Sehr gute Eigenschaften besitzt der Grundkörper 4,6,3',4'-Tetrahydroxybenzylcoumaranon-3. Dies entspricht der Verbindung der Formel I, wobei X = -$CH_2$- und $R^1$ = $R^2$ = $R^3$ = $R^4$ = H ist. Das Potential dieser Verbindung als

Antioxidationsmittel und Radikalfänger, insbesondere bei einer Verwendung in Kosmetika, Pharmazeutika und Nahrungsmitteln, wurde bisher nicht erkannt. Dies trifft auch für das 4, 6, 3', 4'-Tetrahydroxyauron zu, das einer Verbindung der Formel I entspricht, wobei X = =CH- und $R^1 = R^2 = R^3 = R^4$ = H ist. Dies öffnet den Weg zu einer neuen Klasse von Antioxidanzien, die sich durch Variation der Substituenten $R^1$, $R^2$, $R^3$ und $R^4$ ergibt. So kann beispielsweise die Löslichkeit in Wasser oder Ölen an den jeweiligen Bedarf angepaßt werden, indem ein oder mehrere Protonen der Hydroxygruppen der Grundgerüste beispielsweise durch Alkylgruppen, Cycloalkylgruppen oder - alkenylgruppen ersetzt werden. Diese Substituenten können wiederum in ihren Eigenschaften durch Substituenten wie beispielsweise Hydroxygruppen oder Aminogruppen, die wiederum alkyliert sein können, modifiziert werden. Die Löslichkeit in Wasser kann verbessert werden, indem beispielsweise die Reste $R^1$, $R^2$, $R^3$ und $R^4$ als Sulfat- oder Phosphatgruppen gewählt werden. Besonders geeignet ist ein Gemisch aus Mono-, Di- und Trisulfat, das im weiteren als "sulfatisiertes Coumaranon" bezeichnet wird. Besonders hervorgehoben ist das Trisulfat (X = $-CH_2-$; $R^1 = R^3 = R^4 = SO_3Me$, $R^2$ = H), das durch die folgende Formel dargestellt wird.

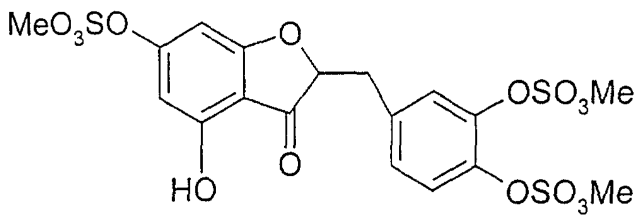

Die Öllöslichkeit lässt sich verbessern, indem beispielsweise die Hydroxygruppen der Grundkörper mit Ethylhexancarbonsäure verestert werden.

Weiter geeignete Reste werden durch Veresterung mit Monocarbonsäuren, wie Buttersäure, Valeriansäure, Hexansäure, Sorbinsäure, Ascorbinsäure oder Laurinsäure erhalten. Durch diese Reste wird der Transport der Verbindung durch biologische Membranen gefördert. In der Zelle kann der Grundkörper dann freigesetzt werden, indem durch Esterasen die Carbonsäuren abgespalten werden.

Eine weitere besonders interessante Klasse von Verbindungen ergibt sich, wenn die Grundkörper, wobei hier vorzugsweise die Grundkörper gemeint sind, in denen X eine Einfachbindung, $-CH_2-$ oder =CH- ist und $R^1 = R^2 = R^3 = R^4$ = H ist, mit UV-absorbierenden Gruppen substituiert sind. Die Verbindungen sowie Formulierungen, die diese Verbindungen enthalten, zeigen dann vorteilhaft eine Schutzwirkung als Antioxidans wie auch als UV-Filter. Bereits die Grundkörper zeigen eine Schutzwirkung gegen UVA-Strahlung. Durch geeignete Reste $R^1$, $R^2$, $R^3$ und $R^4$ können daher UVA/B-Breitbandfilter zur Verfügung gestellt werden.

Die Gruppen werden dabei sinnvoll so ausgewählt, dass sie insbesondere in den für die Haut besonders schädlichen Wellenlängenbereichen der UVA- und der UVB-Strahlung ein Absorptionsmaximum zeigen. Der UV-B-Anteil des Sonnenlichts umfasst den Bereich von 280 bis 320 nm und die UV-A-Strahlung den Bereich von > 320 nm, welcher sich direkt an den Bereich des sichtbaren Lichts anschließt.

Bevorzugt werden auch solche Strukturen ausgewählt, bei denen zumindest einer der Reste $R^1$, $R^2$, $R^3$ und $R^4$ durch ein Mono- oder Oligosaccharid gebildet wird. Bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$-oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.

Auron ($R^1 = R^2 = R^3 = R^4$ = H, X = =CH-) gehört zur Klasse der Bioflavonoide. Unter Bioflavonoiden werden dabei natürlich vorkommende Flavonoide verstanden.

4,6,3',4'-Tetrahydroxybenzylcoumaranon-3 ist beispielsweise aus Quercetin durch Umsetzung von Quercetin mit Natriumdithionit in wässriger Lösung zugänglich. Es unterscheidet sich von Bioflavonoiden durch den Fünfring des Benzofuranons.

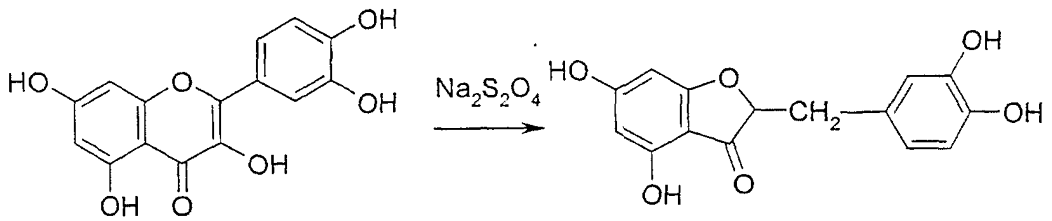


Eine andere Möglichkeit 4,6,3',4'-Tetrahydroxybenzylcoumaranon-3 herzustellen führt, wie in dem britischen Patent GB 2 131 431 beschrieben, über das Auron. Dabei kann auch das Auron künstlich hergestellt werden. Dies ist beispielsweise in dem britischen Patent GB 2 030 142 beschrieben.

Durch Variation der in Formel I mit X bezeichneten Gruppe erschließt sich ein weites Gebiet von Verbindungen. Durch Einbau einer Carbonylgruppe -C(O)- lässt sich die Wellenlänge der UV-Absorption modifizieren. Durch Einbau einer Hydroxy- oder Aminfunktion stehen z.B. weitere Anknüpfungsstellen zur Modifikation der Verbindungen der Formel I zur Verfügung.

In den erfindungsgemäßen Formulierungen ist die Verbindung der Formel I dabei üblicherweise in Mengen von 0,01 - 10 Gew.-% enthalten. Diese Mengen gewährleisten, dass die Verbindung der Formel I bewirkt, dass die erfindungsgemäße Formulierung die angegebenen Eigenschaften aufweisen. Wenn besondere Anforderungen an die Formulierung zu stellen sind, kann die Formulierung jedoch die Verbindung der Formel I auch in abweichenden Mengen enthalten. Besonders bevorzugt ist es, wenn die Verbindung der Formel I in den erfindungsgemäßen Formulierungen in Mengen von 0,02 - 5 Gew.-% enthalten ist, wobei sich Formulierungen wie 0,05 - 0,2 Gew.-% der Verbindung der Formel I enthaltend als besonders vorteilhaft erwiesen haben.

Die schützende Wirkung gegen UV-Strahlung kann verbessert werden, wenn die Formulierung einen oder mehrere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie

- 3-(4'-Methylbenzyliden)-dl-kampfer (z.B. Eusolex® 6300),
- 3-Benzylidenkampfer (z.B. Mexoryl® SD),
- Polymere von N-{(2 und 4)-[(2-oxoborn-3-yliden)methyl]benzyl}acrylamid (z.B. Mexoryl® SW),
- N,N,N-Trimethyl-4-(2-oxoborn-3-ylidenmethyl)anilinium methylsulfat (z.B. Mexoryl® SK) oder
- $\alpha$-(2-Oxoborn-3-yliden)toluol-4-sulfonsäure (z.B. Mexoryl® SL),

Benzoyl- oder Dibenzoylmethane wie

- 1-(4-tert-Butylphenyl)-3-(4-methoxyphenyl)propan-1,3-dion (z.B. Eusolex® 9020) oder
- 4-Isopropyldibenzoylmethan (z.B. Eusolex® 8020),

Benzophenone wie

- 2-Hydroxy-4-methoxybenzophenon (z.B. Eusolex® 4360) oder
- 2-Hydroxy-4-methoxybenzophenon-5-sulfonsäure und ihr Natriumsalz (z.B. Uvinul® MS-40),

Methoxyzimtsäureester wie

- Methoxyzimtsäureoctylester (z.B. Eusolex® 2292),
- 4-Methoxyzimtsäureisopentylester, z.B. als Gemisch der Isomere (z.B. Neo Heliopan® E 1000),

Salicylatderivate wie

- 2-Ethylhexylsalicylat (z.B. Eusolex® OS),
- 4-Isopropylbenzylsalicylat (z.B. Megasol®) oder
- 3,3,5-Trimethylcyclohexylsalicylat (z.B. Eusolex® HMS),
- 4-Aminobenzoesäure und Derivate wie

- 4-Aminobenzoesäure,
- 4-(Dimethylamino)benzoesäure-2-ethylhexylester (z.B. Eusolex® 6007),
- ethoxylierter 4-Aminobenzoesäureethylester (z.B. Uvinul® P25),

und weitere Substanzen wie

- 2-Cyano-3,3-diphenylacrylsäure-2-ethylhexylester (z.B. Eusolex® OCR),
- 2-Phenylbenzimidazol-5-sulfonsäure sowie ihre Kalium-, Natrium- und Triethanolaminsalze (z.B. Eusolex® 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)propenyl) und 0,1 bis 0,4% (methylhydrogen]silylen]] (n ≈ 60) (CAS-Nr. 207574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

Diese organischen UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z. B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxyphenyl)-propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Die Kombination aus den oben genannten UV-Filtern mit einer Verbindung der Formel I in einer Formulierung ergibt dann eine Zusammensetzung, die Lichtschutz mit besonderer Hautfreundlichkeit vereint. Üblicherweise erfolgt die Kombination von UV-Filtern mit Verbindungen der Formel I in solchen Zusammensetzungen in Verhältnissen im Bereich von 100 000:1 bis 1:10, bevorzugt in Mengen von 1000:1 bis 1:10.

Die schützende Wirkung gegen oxidativen Stress bzw. gegen die Einwirkung von Radikalen kann weiter verbessert werden, wenn die Formulierung ein oder mehrere weitere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+) -Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+) -Ascorbylpalmitat und Zitronensäure (z.B. Oxynex® 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Die erfindungsgemäßen Formulierungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (*E. A. Galinski et al., Eur. J. Biochem., 149 (1985) Seite 135-139*). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

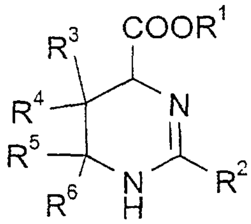

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Formulierungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Formulierungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Formulierungen vorzugsweise 0,01 bis 10 Gew.-% des Aryloxims, wobei es insbesondere bevorzugt ist, wenn die Zubereitung 0,05 bis 5 Gew-% Aryloxim enthält.

Alle Verbindungen oder Komponenten, die in den Formulierungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische Formulierungen eingearbeitet werden. Geeignet sind Formulierungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen kosmetischen oder pharmazeutischen Nahrungsmittelergänzungs-Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Formulierungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

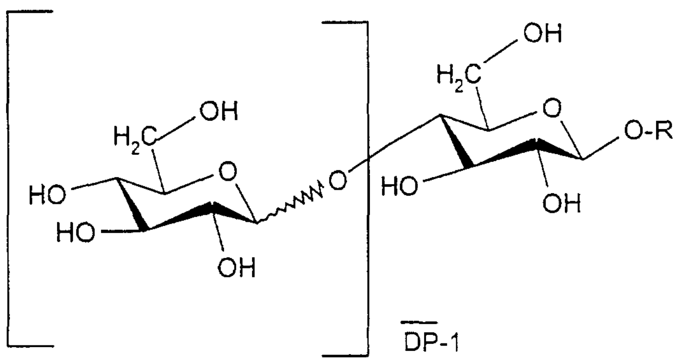

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \Sigma\, \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die

Strukturformel

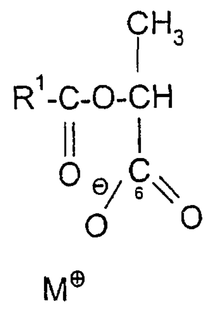

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

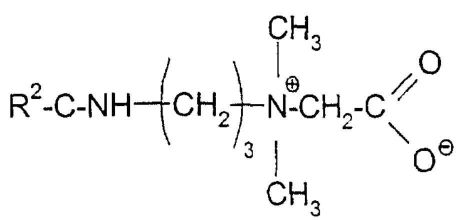

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohienstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitre übliche Coemulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vortil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind:

Polyethylenglycol(13)stearylether (Steareth-13),
Polyethylenglycol(14)stearylether (Steareth-14),
Polyethylenglycol(15)stearylether (Steareth-15),
Polyethylenglycol(16)stearylether (Steareth-16),
Polyethylenglycol(17)stearylether (Steareth-17),
Polyethylenglycol(18)stearylether (Steareth-18),
Polyethylenglycol(19)stearylether (Steareth-19),
Polyethylenglycol(20)stearylether (Steareth-20),
Polyethylenglycol(12)isostearylether (Isosteareth-12),
Polyethylenglycol(13)isostearylether (Isosteareth-13),
Polyethylenglycol(14)isostearylether (Isosteareth-14),
Polyethylenglycol(15)isostearylether (Isosteareth-15),
Polyethylenglycol(16)isostearylether (Isosteareth-16),
Polyethylenglycol(17)isostearylether (Isosteareth-17),
Polyethylenglycol(18)isostearylether (Isosteareth-18),
Polyethylenglycol(19)isostearylether (Isosteareth-19),
Polyethylenglycol(20)isostearylether (Isosteareth-20),
Polyethylenglycol(13)cetylether (Ceteth-13),
Polyethylenglycol(14)cetylether (Ceteth-14),
Polyethylenglycol(15)cetylether (Ceteth-15),
Polyethylenglycol(16)cetylether (Ceteth-16),
Polyethylenglycol(17)cetylether (Ceteth-17),
Polyethylenglycol(18)cetylether (Ceteth-18),
Polyethylenglycol(19)cetylether (Ceteth-19),
Polyethylenglycol(20)cetylether (Ceteth-20),
Polyethylenglycol(13)isocetylether (Isoceteth-13),
Polyethylenglycol(14)isocetylether (Isoceteth-14),
Polyethylenglycol(15)isocetylether (Isoceteth-15),
Polyethylenglycol(16)isocetylether (Isoceteth-16),
Polyethylenglycol(17)isocetylether (Isoceteth-17),
Polyethylenglycol(18)isocetylether (Isoceteth-18),
Polyethylenglycol(19)isocetylether (Isoceteth-19),
Polyethylenglycol(20)isocetylether (Isoceteth-20),
Polyethylenglycol(12)oleylether (Oleth-12),
Polyethylenglycol(13)oleylether (Oleth-13),
Polyethylenglycol(14)oleylether (Oleth-14),
Polyethylenglycol(15)oleylether (Oleth-15),
Polyethylenglycol(12)laurylether (Laureth-1 2),
Polyethylenglycol(12)isolaurylether (Isolaureth-12),
Polyethylenglycol(13)cetylstearylether (Ceteareth-13),
Polyethylenglycol(14)cetylstearylether (Ceteareth-14),
Polyethylenglycol(15)cetylstearylether (Ceteareth-15),
Polyethylenglycol(16)cetylstearylether (Ceteareth-16),
Polyethylenglycol(17)cetylstearylether (Ceteareth-17),
Polyethylenglycol(18)cetylstearylether (Ceteareth-18),
Polyethylenglycol(19)cetylstearylether (Ceteareth-19),
Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,

Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21 )isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol (25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die erfindungsgemäße Zubereitung eignet sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Formulierung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder —milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische oder pharmazeutische Formulierung kann außer der oder den Verbindungen der Formel I verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere Verbindungen der Formel I aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wurde auch festgestellt, dass Verbindungen der Formel I stabilisierend auf die Formulierung wirken können. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind. In einem 2,2-Diphenyl-1-pikrylhydrazyl (DPPH)-Assay konnte gezeigt werden, dass 4,6,3',4'-Tetrahydroxybenzylcoumaranon bessere Radikaifängereigenschaften aufweist als Tocophenol (Vitamin E). Die Durchführung des DPPH-Assays wird beschrieben in Brand-Williams, W., Cuvelier,M.E. & Berset,C. "Use of a free radical method to evaluate antioxidant activity"; *Food Science & Technology* 28, 25-30 (1995). Gamez, E.J. et al. "Antioxidant flavonoid glycosides from Daphniphyllum calycinum"; *J. Nat. Prod.* 61, 706-708 (1998); Ancerewicz, J. et al. "Structure-property relationships of trimetazidine derivates and model compounds as potential antioxidants"; *Free Radic. Biol. Med.* 25, 113-120 (1998).

2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2.2.Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol)

Die Auswertung erfolgt grafisch, indem das molare Verhältnis Testsubstanz/DPPH gegen die prozentuale Extinktionsabnahme aufgetragen und die $EC_{50}$ durch Ablesen bei 50% ermittelt wird. Zusätzlich wird die Steigung der Graden im linearen Bereich ermittelt und der $EC_{50}$ errechnet.

Die positiven Wirkungen von Verbindungen der Formel I ergeben deren besondere Eignung zur Verwendung in kosmetischen oder pharmazeutischen Formulierungen.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre Wirkung als Antioxidationsmittel bzw. als Radikalfänger eignen sich Verbindungen der Formel I, mit Resten, wie oben definiert, wobei -Xsteht für eine Einfachbindung, $-CH_2-$, -C(O)-, $=C(OR^5)-$, $-C(NR^5)-$, $-CH(NR^5R^6)-$ oder $-CH(OR^5)-$, auch als Arzneimittel. Sie wirken dabei unterstützend oder substituierend zu natürlichen Mechanismen, welche Radikale im Körper abfangen. Die Verbindungen der Formel I können in ihrer Wirkung teilweise mit Radikalfängern wie Vitamin C verglichen werden. Verbindungen der Formel I können beispielsweise zur vorbeugenden Behandlungen von Entzündungen und Allergien der Haut sowie in bestimmten Fällen zur Verhütung bestimmter Krebsarten verwendet werden.

Auch Verbindungen der Formel I , wobei -X- steht für =CH- und mindestens ein Rest aus $R^1$, $R^2$, $R^3$ und $R^4$ steht für eine Gruppe, die ausgewählt ist aus der folgenden Liste

- geradkettige oder verzweigte $C_1$- bis $C_{12}$-Alkylcarbonylgruppen,
- geradkettige oder verzweigte $C_3$- bis $C_{12}$-Alkenylcarbonylgruppen,
- geradkettige oder verzweigte $C_1$- bis $C_{12}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- $C_3$- bis $C_{10}$-Cycloalkylcarbonylgruppen und $C_3$- bis $C_{12}$-Cycloalkenyl- und/oder Cycloalkenylcarbonylgruppen, wobei die Ringe jeweils auch durch $-(CH_2)_n$-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste,

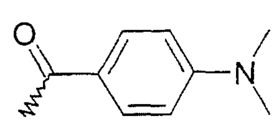

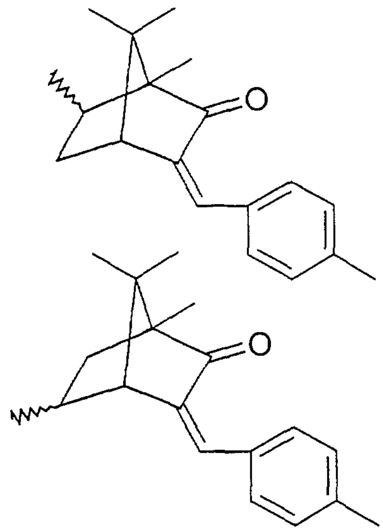

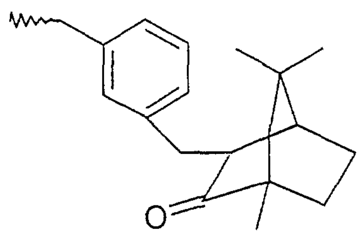

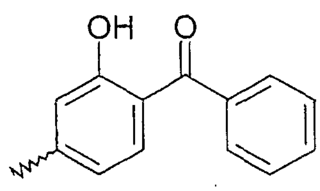

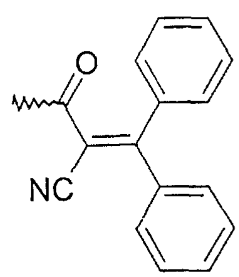

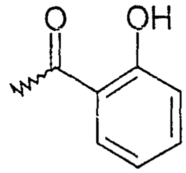

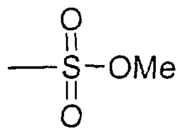

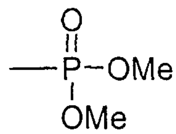

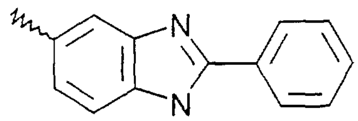

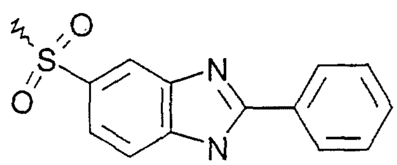

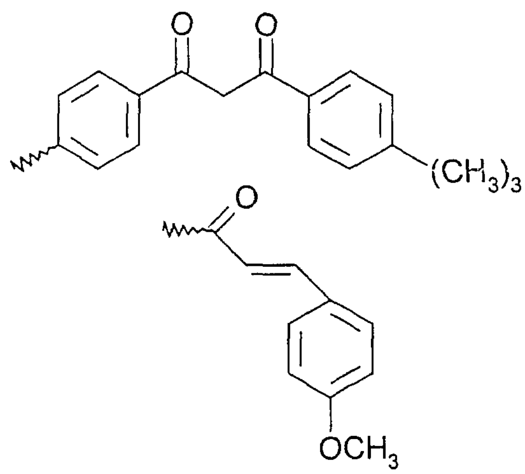

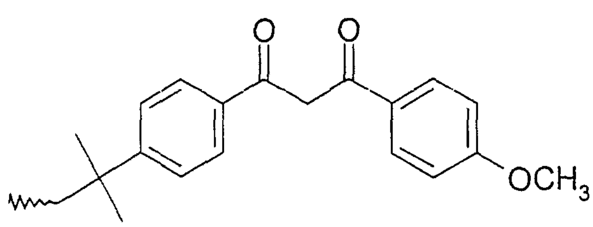

wobei Me steht für ein Proton oder ein Alkalimetallion, insbesondere ein Natrium- oder Kaliumion, eignen sich aufgrund der oben angeführten Eigenschaften als Arzneimittel.

Insbesondere eignen sich Verbindungen der Formel I zur Herstellung eines Arzneimittels zur Behandlung

von Entzündungen, Allergien und Irritationen, insbesondere der Haut.

Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel.

Weiter zeigen Verbindungen der Formel I antiallergische und antiinflammatorische und antiirritative Wirkungen. Sie eignen sich daher zur Herstellung von Arzneimitteln zur Behandlung von Entzündungen oder allergischen Reaktionen.

Weiterhin sind Gegenstand der Erfindung Verbindungen der Formel I.

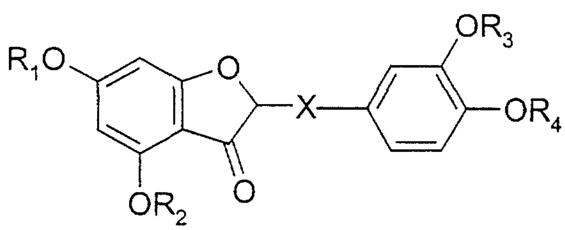

I

wobei die Variablen die oben angegebene Bedeutung aufweisen, die dadurch kennzeichnet sind, dass mindestens ein Rest aus $R^1$, $R^2$, $R^3$ und $R^4$ nicht H ist.

Durch die Reste $R^1$, $R^2$, $R^3$ und $R^4$ lassen sich die Eigenschaften der Verbindungen nahezu beliebig auf die jeweiligen Bedürfnisse abstimmen. So kann durch die Einführung beispielsweise von Alkylketten die Löslichkeit der Verbindungen in Ölen verbessert werden. Durch die Einführung von UV-absorbierenden Gruppen lassen sich Verbindungen darstellen, die sowohl Antioxidanzien bzw. Radikalfänger sind ais auch UV-Absorbenzien. Die Einführung der Reste kann zum Beispiel ausgehend von den bekannten Verbindungen 4,6,3',4'-Tetrahydroxybenzylcoumaranon-3 (X = $-CH_2-$) bzw. 4,6,3',4'-Tetrahydroxyauron (X = =CH-) erfolgen durch dem Fachmann an sich bekannte Verfahren. So können Alkylketten durch Reaktion der Grundkörper mit dem entsprechenden Alkylhalogeniden unter Wirkung einer starken Base in einem geeigneten Lösungsmittel, beispielsweise Dimethylformamid, eingeführt werden. Erfolgt eine Anbindung der Reste $R^1$ bis $R^4$ als Ester, kann dies beispielsweise durch Umsetzung mit einem entsprechenden Säurechlorid erfolgen. Allgemeine Herstellverfahren sind beispielsweise in Houben-Weyl; Georg Thieme Verlag Stuttgart, beschrieben.

Stellvertretend sind im folgenden einige geeignete Reaktionen genannt:

Anknüpfung durch Esterbildung: Houben-Weyl; Band VIII, S. 503ff.; Herausgeber: E. Müller. Houben-Weyl; Band E5, S. 656ff.; Herausgeber: J. Falbe.

Anknüpfung durch Etherbildung: Houben-Weyl; Band VI/3, S. 1 ff.;

Herausgeber: E. Müller.

Anknüpfung durch Sulfonsäureester: Houben-Weyl; Band IX; S. 659 ff.; Herausgeber: E. Müller.

Stellvertretend sind in Schema 1 einige Vorstufen der Reste R angegeben, die für eine Einführung der Reste in den Grundkörper geeignet sind. Diese Aufstellung ist nur beispielhaft und zeigt nur einen kleinen Ausschnitt aus der Menge der möglichen Vorstufen. Dem Fachmann ist es ohne weiteres möglich, andere geeignete Abgangsgruppen, beispielsweise eine Tosylatgruppe oder eine Triflatgruppe anstelle der Halogenide vorzusehen. Beispielsweise ist eine Einführung der Reste über eine Esterfunktion auch durch entsprechende Umesterungsreaktionen möglich.

Schema 1: Beispiele für geeignete Vorstufen zur Einführung von Resten $R^1$, $R^2$, $R^3$, $R^4$:

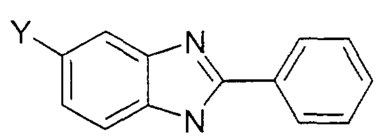

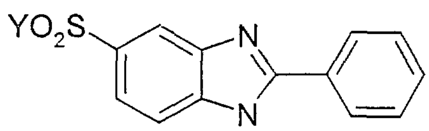

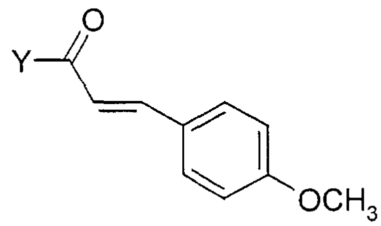

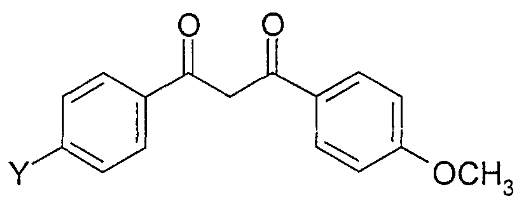

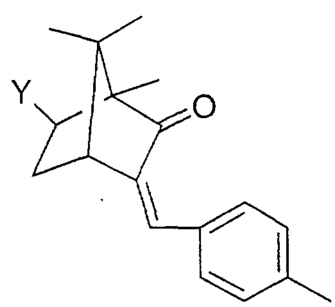

Y steht dabei für eine geeignete Abgangsgruppe.

Die Verbindungen können entweder als reine Verbindungen vorliegen, d.h. dass beispielsweise nur bestimmte Reste von den oben angegebenen Gruppen gebildet werden, während die anderen Reste Wasserstoff sind. Es ist jedoch auch möglich, dass die Reste statistisch verteilt sind, also beispielsweise ein Isomerengemisch vorliegt oder ein Gemisch von Verbindungen, bei denen 1, 2, 3 oder 4 Wasserstoffatome des Grundkörpers durch einen oder mehrere der oben angegebenen Reste ersetzt sind.

Zu den erfindungsgemäß bevorzugten Verbindungen gehören dabei die Verbindungen, bei denen mindestens ein Rest aus $R^1$, $R^2$, $R^3$ und $R^4$ eine Gruppe $-SO_3Me$ oder $-PO_3Me_2$ ist. Diese Verbindungen besitzen ein im Vergleich zu den Hydroxyverbindungen verbesserte Wasserlöslichkeit und lassen sich daher in wasserhaltige Formulierungen gut einarbeiten. Besonders bevorzugt sind dabei die Sulfate, insbesondere die Trisulfate gemäß Formel III

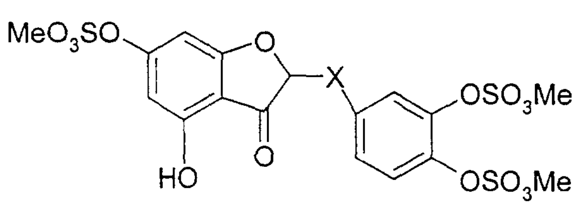

III

die rein oder in Mischung mit den entsprechenden Mono- und Disulfaten vorliegen können. Insbesondere bevorzugt

ist das Sulfat, insbesondere das Trisulfat von 4,6,3',4'-Tetrahydroxybenzylcoumaranon-3,

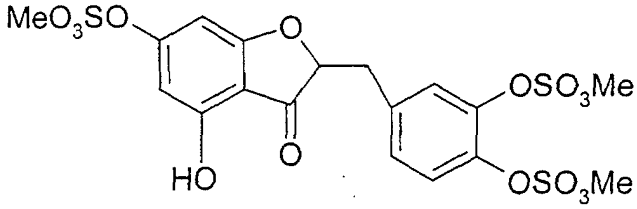

das vorzugsweise in Form eines löslichen Salzes, vorzugsweise eines Alkalimetallsalzes eingesetzt wird, und entweder rein, oder in einer weiteren bevorzugten Ausführungsform im Gemisch mit Mono- und Disulfat vorliegt.

Erfindungsgemäß insbesondere bevorzugt sind Verbindungen der Formel I, wobei -X- steht für eine Einfachbindung, $-CH_2-$, -C(O)-, $=C(OR^5)-$, $-C(NR^5)-$, $-CH(NR^5R^6)-$ oder $-CH(OR^5)-$.

Die Umsetzung der Grundkörper mit den entsprechenden reaktiven Vorstufen der Reste $R^1$ bis $R^4$ erfolgt nach dem Fachmann bekannten Verfahren und in den üblichen Lösungsmitteln. Geeignete Lösungsmittel sind beispielsweise chlorierte Kohlenwasserstoffe, Dimethylsulfoxid, Dimethylformamid usw.

Die Verwendung einer erfindungsgemäß bevorzugten Verbindung der Formel I, wobei -X- steht für eine Einfachbindung, $-CH_2-$, -C(O)-, $=C(OR^5)-$, $-C(NR^5)-$, $-CH(NR^5R^6)-$ oder $-CH(OR^5)-$ und die übrigen Variablen die allgemeine obenangegebene Bedeutung aufweisen, als Antioxidationsmittel, insbesondere für kosmetische Formulierungen oder Nahrungsmittel ist ein weiterer Gegenstand der vorliegenden Erfindung.

Ein weiterer Gegenstand der Erfindung betrifft die Stabilisierung von UV-Filtern. Eine bekannte und leistungsfähige Klasse von Lichtschutzfiltersubstanzen wird von den Dibenzoylmethanderivaten gebildet. Nachteilig ist jedoch, dass diese Substanzen sehr leicht durch UV-Licht zersetzt werden und damit ihre schützenden Eigenschaften verlorengehen. Als Beispiel für einen auf dem Markt erhältlichen Lichtschutzfilter aus dieser Verbindungsklasse sei das 4-(tert.-Butyl)-4'-methoxydibenzoylmethan angeführt, welches die unten gezeigte Struktur aufweist.

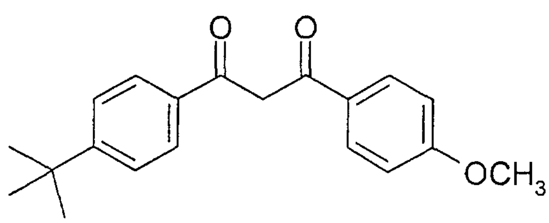

Überraschend hat sich nun gezeigt, dass Verbindungen mit der Formel I, wobei -X- steht für eine Einfachbindung, $-CH_2-$, -C(O)-, $=C(OR^5)-$, $-C(NR^5)-$, $-CH(NR^5R^6)-$ oder $-CH(OR^5)-$ eine sehr gute Stabilisierungswirkung für die Dibenzoylmethane, insbesondere 4-(tert.-buthyl)-4-methoxibenzoylmethan, aufweisen. Eine besonders hohe Stabilisierungswirkung wurde für eine Verbindung der Formel I gefunden, wobei $X = -CH_2-$ und $R^1 = R^2 = R^3 = R^4 = H$ ist. Indem Gemische dieser Verbindungen in Kosmetika eingearbeitet werden, ist es nun möglich, Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

Die Erfindung wird anhand von Beispielen und unter Bezugnahme auf eine Zeichnung näher erläutert.

In den im folgenden aufgeführten Rezepturen wird unter "sulfatisiertem Coumaranon, Kaliumsalz" bzw. THBC-Sulfat ein Gemisch aus Mono-, Di- und Trisulfat des 4,6,3', 4'-Tetrahydroxybenzylcoumaranon-3 verstanden, wobei die Verbindungen des Gemischs als Kaliumsalz vorliegen.

Antaron® V-220 wird von der Fa. GAF, Frechen, DE, vertrieben.

Carbomer Ultrez-1 0 wird von der Fa. Goodrich, Neuss, DE, angeboten.

Dehymuls® E ist eine Mischung aus Dicocolypentaerytritylcitrat, Sorbitolsesquioleat, Bienenwachs und Aluminiumstearat und wird von der Henkel KGaA, Düsseldorf, DE, vertrieben.

Eusolex® 2292, Eusolex® HMS, Eusolex® 6300 und Eusolex® 232 sind UV-Filter, die von der Merck KGaA, Darmstadt, vertrieben werden.

HMLO steht für 5-Hydroxy-5-methyllaurophenonoxim

| | |
|---|---|
| Luvitol® EHO | wird von der Fa. BASF AG, Ludwigshafen, DE, vertrieben, |
| MBTTBP | 2,2'-Methylen-bis-(6-2H-benzotriazol-2-yl)-4-(1,1,3,3-tetramethylbutyl)-phenol |
| Pemulen® TR-1 | ist ein Acrylat/C-C-Alkylacrylatpolymeres, das von der Firma Goddrich, Neuss, DE, vertrieben wird, |
| Pemulen®TR-2 | ist ein Acrylat/$C_{10}$-$C_{30}$-Alkylacrylatpolymeres, das von der Fa. Goodrick, Neuss, vertrieben wird, |
| Performa® V825 | ist ein synthetisches Wachs, das von der Fa. New Phase, NJ08554 vertrieben wird, |
| Oxynex® K | ist eine Mischung aus PEG-8, Tocophenol, Ascorbylpalmitat, Ascorbinsäure und Zitronensäure und wird von der Merck KGaA, Darmstadt vertrieben. |
| Uvasorb® HEB | ist ein Dioctylbutamidotriazon und wird von der Firma 3V Sigma vertrieben. |

**Beispiel 1**

| | Pflegelotion (W/O) zum Auftragen auf die Haut | | Gew.-% |
|---|---|---|---|
| A | 4,6,3',4'-Tetrahydroxybenzylcoumaranon-3 | | 1,00 |
| | Polyglyceryl-2-Dipolyhydroxystearat | | 5,00 |
| | Bienenwachs | | 0,50 |
| | Zinkstearat | | 0,50 |
| | Hexyllaurat | | 9,00 |
| | Cerylisononanoat | | 6,00 |
| | Shea Butter | | 0,50 |
| | DL-α-Tocopherolacetat | | 1,00 |
| B | Glycerin | | 5,00 |
| | Magnesiumsulfat-Heptahydrat | | 1,00 |
| | Konservierungsmittel | | q.s. |
| | Wasser, demineralisiert | Aqua ad | 100,00 |

**Herstellung:**

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 2**

| Pflegelotion (W/O) zum Auftragen auf die Haut | | Gew.-% |
|---|---|---|
| A | Polyglyceryl-2-dipolyhydroxystearat | 5,00 |
| | Bienenwachs | 0,50 |
| | Zinkstearat | 0,50 |

(fortgesetzt)

| Pflegelotion (W/O) zum Auftragen auf die Haut | | Gew.-% |
|---|---|---|
| | Hexyllaurat | 9,00 |
| | Cerylisononanoat | 6,00 |
| | Shea Butter | 0,50 |
| | DL-α-Tocopherolacetat | 1,00 |
| B | sulfatisiertes Coumaranon, Kaliumsalz | 0,50 |
| | Glycerin | 5,00 |
| | Magnesiumsulfat-Heptahydrat | 1,00 |
| | Konservierungsmittel | q.s. |
| | Wasser, demineralisiert | ad 100,00 |

**Herstellung:**

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 3**

| Hautcreme (O/W) | | Gew.% |
|---|---|---|
| A | Paraffin | 2,00 |
| | Isohexadecan | 2,00 |
| | Isopropylpalmitat | 3,00 |
| | Sojaöl | 0,50 |
| | Dimeticon | 1,00 |
| | Ceryl Alkohol | 1,00 |
| | Sorbitolstearat | 1,50 |
| | Cerylalkohol (und) Cerylglucosid | 4,00 |
| | (-) -α- Bisabolol | 0,30 |
| B | Wasser, demineralisiert | ad 100,00 |
| | sulfatisiertes Coumaranon, Kaliumsalz | 0,20 |
| | Glycerin 87 % | 10,00 |
| | D-Panthenol | 0,50 |
| | (D+)-Biotin | 0,05 |
| | Konservierungsmittel | q.s. |
| C | Xanthan Gummi | 0,30 |
| D | Parfüm | 0,20 |

**Herstellung:**

Phase A und Phase B werden getrennt auf 75°C erwärmt. Dann wird Phase C langsam zu Phase B gegeben und bis zur Homogenisierung gerührt. Die erhaltene Mischung B/C wird bei einer Temperatur von 75°C zu Phase A

gegeben und die Mischung homogenisiert. Nach Abkühlen auf 35°C werden Parfümstoffe zugegeben.

Als Konservierungsmittel werden verwendet 0,05 % Propyl-4-hydroxybenzoat, 0,15 % Methyl-4-hydroxybenzoat und 0,30 % Germall 115 (ISP, Frechen).

**Beispiel 4**

| | | Gew.% |
|---|---|---|
| A | 4,6,3',4'-Tetrahydroxybenzylcoumaranon-3 | 0,10 |
| | Paraffin | 2,00 |
| | Isohexadecan | 2,00 |
| | Isopropylpalmitat | 3,00 |
| | Sojaöl | 0,50 |
| | Dimeticon | 1,00 |
| | Cerylalkohol | 1,00 |
| | Sorbitolstearat | 1,50 |
| | Cerylalkohol (und) Cerylglucosid | 4,00 |
| | (-) -α- Bisabolol | 0,30 |
| B | Wasser, demineralisiert | ad 100,00 |
| | Glycerin 87 % | 10,00 |
| | D-Panthenol | 0,50 |
| | (D+)-Biotin | 0,05 |
| | Konservierungsmittel | q.s. |
| C | Xanthan Gummi | 0,30 |
| D | Parfüm | 0,20 |

**Herstellung:**

Phase A und Phase B werden getrennt auf 75°C erwärmt. Dann wird Phase C langsam zu Phase B gegeben und bis zur Homogenisierung gerührt. Die erhaltene Mischung B/C wird bei einer Temperatur von 75°C zu Phase A gegeben und die Mischung homogenisiert. Nach Abkühlen auf 35°C werden Parfümstoffe zugegeben.

Als Konservierungsmittel werden verwendet:

0,05 % Propyl-4-hydroxybenzoat
0,15 % Methyl-4-hydroxybenzoat
0,30 % Germall 115 (ISP, Frechen)

**Beispiel 5**

| Sonnenschutzspray (O/W) | | Gew.-% |
|---|---|---|
| A | 4,6,3',4'-Tetrahydroxybenzylcoumaranon-3 | 0,50 |
| | Eusolex®2292 (Art.-Nr. 105382) | 7,50 |
| | Eusolex®HMS (Art.-Nr. 111412) | 7,00 |
| | Steareth-2 | 0,40 |
| | Steareth-10 | 0,80 |
| | Pemulen® TR-2 | 0,18 |
| | Propylen-Glycol Isoceteth-3 Acetat | 5,00 |

(fortgesetzt)

| Sonnenschutzspray (O/W) | | Gew.-% |
|---|---|---|
| | Performa® V 825 | 0,80 |
| | Dimeticon | 1,00 |
| | Oxynex®K (Art.-Nr. 108324) | 0,10 |
| | | |
| B | Eusolex®232 (Art. Nr. 105372) | 1,00 |
| | Triethanolamin | 0,90 |
| | Propandiol-1,2 | 2,00 |
| | Konservierungsmittel | 0,50 |
| | Wasser demineralisiert | a.d. 100,00 |

**Herstellung:**

Phase B:

Das Wasser wird mit dem Triethanolamin vermischt und anschließend Eusolex® 232 unter Rühren zugegeben. Sobald sich alles gelöst hat, werden die weiteren Bestandteile der Phase B zugegeben und anschließend die Mischung auf 80°C erwärmt.

Phase A:

Die Bestandteile der Phase A mit Ausnahme von Permulen® TR-2 werden zusammengegeben und auf 80°C erwärmt. Anschließend wird das Permulen® TR-2 unter Rühren zugegeben.

Herstellung des Sonnenschutzmittels:

Zur Phase A wird unter Rühren langsam die Phase B gegeben. Nach dem Homogenisieren wird unter Rühren abgekühlt.

Als Konservierungsmittel wurde verwendet:

0,05 % Propyl-4-hydroxybenzoat
0,15 % Methyl-4-hydroxybenzoat

**Beispiel 6**

| Sonnenschutzspray (O/W) | | Gew.-% |
|---|---|---|
| A | Eusolex®2292 (Art.-Nr. 105382) | 7,50 |
| | Eusolex®HMS (Art.-Nr. 111412) | 7,00 |
| | Steareth-2 | 0,40 |
| | Steareth-10 | 0,80 |
| | Pemulen® TR-2 | 0,18 |
| | Propylen-Glycolisoceteth-3-acetat | 5,00 |
| | Performa® V 825 | 0,80 |
| | Dimeticon | 1,00 |
| | Oxynex®K (Art.-Nr. 108324) | 0,10 |
| | | |
| B | sulfatisiertes Coumaranon, Kaliumsalz | 0,20 |
| | Eusolex®232 (Art.-Nr. 105372) | 1,00 |
| | Triethanolamin | 0,90 |
| | Propandiol-1,2 | 2,00 |

(fortgesetzt)

| Sonnenschutzspray (O/W) | | Gew.-% |
|---|---|---|
| | Wasser, demineralisiert | Aqua ad 100,00 |

**Herstellung:**

Phase B:

Das Wasser wird mit dem Triethanolamin vermischt und anschließend Eusolex® 232 unter Rühren zugegeben. Sobald sich alles gelöst hat, werden die weiteren Bestandteile der Phase B zugegeben und anschließend die Mischung auf 80°C erwärmt.

Phase A:

Die Bestandteile der Phase A mit Ausnahme von Permulen® TR-2 werden zusammengegeben und auf 80°C erwärmt. Anschließend wird das Permulen® TR-2 unter Rühren zugegeben.

Herstellung des Sonnenschutzmittels:

Zur Phase A wird unter Rühren langsam die Phase B gegeben. Nach dem Homogenisieren wird unter Rühren abgekühlt.
Als Konservierungsmittel werden verwendet:

0,05 % Propyl-4-hydroxybenzoat
0,15 % Methyl-4-hydroxybenzoat

**Beispiel 7**

| Sonnenschutzgel (wässrig) | | **Gew.-%** |
|---|---|---|
| **A** | 4,6,3',4'-Tetrahydroxybenzylcoumaranon-3 | 0,10 |
| | Eusolex®232 (Art.-Nr. 105372) | 4,00 |
| | Natriumhydroxidlösung | 6,00 |
| | Glycerin | 3,00 |
| | Propandiol-1,2 | 2,00 |
| | Konservierungsmittel | q.s. |
| | Wasser, demineralisiert | ad 100,00 |
| **B** | Carbomer Ultrez-10 | 0,70 |
| | Wasser, demineralisiert | 60,00 |
| **C** | Natriumhydroxidlösung (10 %) | 1,50 |
| | Wasser, demineralisiert | 4,00 |

**Herstellung:**

Carbomer Ultrez-10 wird im Wasser der Phase B vollständig dispergiert. Anschließend wird langsam die Phase C zugegeben und homogenisiert. Für die Phase A wird zunächst das Wasser zur Natriumhydroxidlösung gegeben und dann das Eusolex® 232 zugegeben und unter Rühren vollständig gelöst. Nach Erhalt einer klaren Lösung werden die weiteren Bestandteile der Phase A zugegeben. Anschließend wird Phase A portionsweise zum Gemisch der Phasen B und C gegeben, wobei nach jeder Zugabe homogenisiert wird.
Als Konservierungsmittel wird verwendet:

0,20 % Methyl-4-hydroxybenzoat

**Beispiel 8**

| Sonnenschutzgel (O/W) | | Gew.-% |
|---|---|---|
| **A** | 4,6,3',4'-Tetrahydroxybenzylcoumaranon-3 | 0,10 |
| | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K | 1,00 |
| **B** | Eusolex® 232 (Art. Nr. 5372) | 0,75 |
| | Tris(hydroxymethyl)-aminomethan | 0,33 |
| | Konservierungsmittel | q.s. |
| | Wasser, demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

**Herstellung:**

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung weden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.

Als Konservierungsmittel werden verwendet:

0,05 % Propyl-4-hydroxybenzoat
0,15 % Methyl-4-hydroxybenzoat

**Beispiel 9**

| Sonnenschutzgel (O/W) | | Gew.-% |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K liquid (Art.-Nr. 8324) | 1,00 |
| **B** | sulfatisiertes Coumaranon, Kaliumsalz | 1,00 |
| | Eusolex® 232 (Art. Nr. 5372) | 0,75 |

(fortgesetzt)

| Sonnenschutzgel (O/W) | | Gew.-% |
|---|---|---|
| | Tris(hydroxymethyl)-aminomethan | 0,33 |
| | Konservierungsmittel | q.s. |
| | Wasser, demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

**Herstellung:**

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung weden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.

Als Konservierungsmittel wird verwendet 0,05 % Propyl-4-hydroxybenzoat und 0,15 % Methyl-4-hydroxybenzoat.

**Beispiel 10**

| Sonnenschutzlotion (W/O) mit UVA/B-Schutz | | Gew.-% |
|---|---|---|
| **A** | 4,6,3',4'-Tetrahydroxybenzylcoumaranon-3 | 0,05 |
| | Eusolex® 2292 (Art-Nr. 1.05382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dicaprylether | 5,00 |
| | Dimeticon | 1,00 |
| **B** | Glycerin (87 %) | 5,00 |
| | Magnesiumsulfate Heptahydrat | 1,00 |
| | Konservierungsmittel | q.s. |
| | Wasser, demineralisiert | ad 100,00 |

**Herstellung:**

Die Bestandteile der Phasen A und B werden jeweils vermengt. Phase A wird auf 75°C und Phase B getrennt auf 80°C erwärmt. Phase B wird unter Homogenisieren zu Phase A gegeben. Anschließend wird unter Rühren abgekühlt.

Als Konservierungsmittel werden verwendet:

0,05 % Propyl-4-hydroxybenzoate
0,15 % Methyl-4-hydroxybenzoate.

**Beispiel 11**

| Sonnenschutzlotion (W/O) mit UVA/B-Schutz | | Gew.-% |
|---|---|---|
| **A** | Eusolex® 2292 (Art-Nr. 1.05382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dicaprylether | 5,00 |
| | Dimeticon | 1,00 |
| **B** | sulfatisiertes Coumaranon, Kaliumsalz | 0,50 |
| | Glycerin (87 %) | 5,00 |
| | Magnesiumsulfate Heptahydrate | 1,00 |
| | Konservierungsmittel | q.s. |
| | Wasser, demineralisiert | ad 100,00 |

**Herstellung:**

Die Bestandteile der Phasen A und B werden jeweils vermengt. Phase A wird auf 75°C erwärmt und getrennt davon Phase B auf 80°C. Phase B wird unter Homogenisierung zu Phase A gegeben. Anschließend wird unter Rühren abgekühlt.

Als Konservierungsmittel werden verwendet:

0,05 % Propyl-4-hydroxybenzoat
0,15 % Methyl-4-hydroxybenzoat.

**Beispiel 12**

**Herstellung von 4,6,3',4'-Tetrahydroxycoumaranon-3**

10 g Quercetin werden portionsweise in 1 l siedendes Wasser eingetragen. Anschließend werden 85 g Natriumcarbonat und 200 g Natriumdithimit zugegeben und die Suspension 25 Minuten bei 100°C gerührt. Dann wurde die Mischung auf 5°C abgekühlt und langsam 130 ml rauchende Salzsäure (37 %) zugetropft. Es wird noch weitere 2 h in der Kälte gerührt und anschließend filtriert. Der Rückstand 2,6 g besteht aus 4,6,3', 4'-Tetrahydroxycoumaranon, das noch mit Quercetin verunreinigt ist.

Das Filtrat wird zunächst mit 700 ml und anschließend mit 200 ml Ethylacetat extrahiert. Die vereinigten organischen Phasen werden mit Wasser (200 ml), gesättigter NACI-Lösung (200 ml) gewaschen und über Natriumsulfat getrocknet. Nach Filtration wird das Lösungsmittel unter vermindertem Druck abdestilliert. Es wird ein gelber Feststoff (4,53 g) erhalten.

**Beispiel 13**

**Herstellung von sulfatisiertem 4,6,3',4'-Tetrahydroxybenzyl-coumaranon-3**

23,8 g Tetrabutylammoniumhydrogensulfat und 21,7 g N, N'-Dicyclohexylcarbodiimid wurden in 250 ml trokkenem Pyridin gelöst. Dann wurden 10 g 4,6,3',4'-Tetrahydroxybenzylcoumaranon-3 zugegeben und das Gemisch über Nacht bei 50 °C gerührt. Nach Abkühlen auf Raumtemperatur wurde filtriert und das Filtrat mit 250 ml Methanol versetzt. Nach Stehen über Nacht wurde erneut vom ausgefallenem Niederschlag abfiltriert, das Filtrat unter vermindertem Druck auf 150 ml eingeengt und erneut vom ausgefallenen Niederschlag abfiltriert. Zum Filtrat wurde eine Lösung von 1,5 g Kaliumcarbonat in 250 ml Methanol gegeben. Nachdem die Mischung mit 5 ml Wasser versetzt

worden war, wurde die Mischung in zwei Zentrifugengläser überführt und zentrifugiert. Die flüssige Phase wurde abdekantiert und der feste Rückstand im Vakuumtrockenschrank getrocknet.

Ausbeute: 2,23 g hellbraunes Pulver, Gemisch von Tri-, Di- und Monosulfat.

**Beispiel 14**

Bestimmung des $EC_{50}$ von 4,6,3', 4'-Tetrahydroxybenzylcoumaranon mit dem DPPH-Assay

Es wurde eine 70 µm Stammlösung von 2,2-Diphenyl-1-pikrylhydrozyl (DPPH) in Ethanol hergestellt. Aliquots dieser Lösung wurden jeweils mit einer solchen Menge an 4.6.3', 4'-Tetrahydroxbenzylcoumaranon (THBC) versetzt, dass die in Tabelle 1 angegebenen Verhältnisse THBC/DPPH erhalten wurden. Es wurde bei 515 nm, 25°C und 1 cm jeweils die Extinktion gemessen, wobei in Tabelle 1 jeweils der Wert angegeben ist, bei dem eine konstante Extinktion gemessen wurde. Die Werte sind in Fig. 1 graphisch gegeneinander aufgetragen, wobei auf der x-Achse das molare Verhältnis und auf der y-Achse die gemessene Extinktion als relativer Anteil der für die Stammlösung gemessenen Extinktion (100 %) aufgetragen ist.

Als $EC_{50}$ wurde 0,17 ermittelt. Die eingesetzte Konzentration der Testsubstanz bei dem $EC_{50}$-Wert betrug 8,7 µmol.

Mit dem gleichen Versuchsaufbau wurde für Tocopherol ein $EC_{50}$ von 0,21 ermittelt. 4,6,3',4'-Tetrahydroxycoumaranon ist damit ein potenteres Antioxidans als Tocopherol (Vitamin E).

Tabelle 1:

| **Extinktionsbestimmung beim DPPH-Assay** | |
|---|---|
| *THBC/DPPH* | **Extinktion in % bezogen auf Extinktion ohne THBC** |
| 0 | 100 |
| 0,01 | 97,66 |
| 0,02 | 94,04 |
| 0,05 | 87,02 |
| 0,07 | 79,15 |
| 0,10 | 72,13 |
| 0,12 | 62,72 |
| 0,18 | 44,18 |
| 0,24 | 26,08 |
| 0,30 | 16,16 |
| 0,37 | 15,73 |
| 0,43 | 15,27 |
| 0,49 | 15,27 |
| 0,55 | 15,27 |
| 0,61 | 15,05 |
| 0,73 | 14,19 |
| 0,97 | 14,56 |
| 1,21 | 14,56 |

**Beispiel 15**

| O/W-Emulsion | Gew.-% |
|---|---|
| Stearinsäure | 1,50 |
| Glycerylstearat SE | 3,50 |
| Caprylsäure/Caprinsäuretriglycerid | 5,00 |
| Octyldodecanol | 5,00 |
| Dicaprylether | 5,00 |
| Cetylstearylalcohol | 0,50 |
| Xanthangummi | 0,50 |
| Octyltriazon | 0,50 |
| MBTTBP | 6,00 |
| Titandioxid | 2,00 |
| $\alpha$-Glucosylrutin | 0,20 |
| Glycerin | 3,00 |
| Natronlauge 45 % | 0,03 |
| THBC | 0,10 |
| Ectoin | 1,00 |
| Konservierung | 0,50 |
| Wasser | ad 100,00 |

**Beispiel 16**

| O/W-Emulsion | Gew.-% |
|---|---|
| Stearinsäure | 1,50 |
| Glycerylstearat SE | 3,50 |
| Caprylsäure/Caprinsäuretriglycerid | 9,00 |
| Octyldodecanol | 9,00 |
| Dicaprylylether | 9,00 |
| Cetylstearylalcohol | 0,50 |
| Xanthangummi | 0,50 |
| Dibenzoylmethan | 2,00 |
| Methylbenzylidencampher | 4,00 |
| Bornitrid | 0,50 |
| $\alpha$-Glucosylrutin | 0,50 |
| Glycerin | 5,00 |
| Natronlauge 45 % | 0,03 |
| THBC | 0,20 |
| HMLO | 0,30 |
| EDTA-Lösung | 1,00 |
| Konservierung | 0,50 |
| Wasser | ad 100,00 |

**Beispiel 17**

| O/W-Emulsion | Gew.-% |
|---|---|
| Sorbitanstearat | 2,00 |
| Polyglyceryl-3-methylglucosedistearat | 4,00 |
| Caprylsäure/Caprinsäuretriglycerid | 5,00 |
| Octyldodecanol | 5,00 |
| Dicaprylylether | 5,00 |
| Xanthangummi | 0,50 |
| Octyltriazon | 2,00 |
| Uvasorb HEB | 8,00 |
| THBC | 0,35 |
| HMLO | 0,10 |
| Glycerin | 3,00 |
| Phenylbenzimidazolsulfonsäure | 2,00 |
| Natronlauge 45 % | 0,70 |
| Ectoin | 2,00 |
| Konservierung | 0,50 |
| Wasser | ad 100,00 |

**Beispiel 18**

| O/W-Emulsion | Gew.-% |
|---|---|
| Sorbitanstearat | 2,00 |
| Polyglyceryl-3-methylglucosedistearat | 4,00 |
| Caprylsäure/Caprinsäuretriglycerid | 5,00 |
| Octyldodecanol | 5,00 |
| Dicaprylylether | 5,00 |
| Xanthangummi | 0,50 |
| Dibenzoylmethan | 2,00 |
| Methylbenzylidencampher | 4,00 |
| THBC-Sulfat | 0,20 |
| Bornitrid | 1,00 |
| Ectoin | 2,00 |
| Glycerin | 5,00 |
| Phenylbenzimidazolsulfonsäure | 1,00 |
| Natronlauge 45 % | 0,35 |
| EDTA-Lösung | 1,00 |
| Konservierung | 0,50 |
| Wasser | ad 100,00 |

**Beispiel 19**

| O/W-Emulsion | Gew.-% |
|---|---|
| Butylenglycoldicaprylat/Dicaproat | 10,00 |
| Sheabutter | 0,50 |

(fortgesetzt)

| O/W-Emulsion | Gew.-% |
|---|---|
| Phenyltrimethicon | 2,00 |
| Acrylat/$C_{10}$-$_{30}$-Alkylacrylat Crosspolymer | 0,50 |
| Xanthangummi | 0,50 |
| T 150 | 3,00 |
| α-Glucosylrutin | 1,20 |
| THBC-Sulfat | 0,30 |
| Ectoin | 0,10 |
| Glycerin | 3,00 |
| Citronensäure | 0,40 |
| Natronlauge 45 % | 0,15 |
| Konservierung | 0,50 |
| Wasser | ad 100,00 |

**Beispiel 20**

| Hydrodispersion | Gew.-% |
|---|---|
| ButylenglycoldicaprylatlDicaproat | 10,00 |
| Sheabutter | 0,50 |
| Phenyltrimethicon | 2,00 |
| Acrylat/$_{10\text{-}30}$-Alkylacrylat Crosspolymer | 0,50 |
| Xanthangummi | 0,50 |
| Dibenzoylmethan | 1,00 |
| Methylbenzylidencampher | 2,00 |
| Uvasorb HEB | 4,00 |
| THBC | 0,30 |
| 5-Hydroxyectoin | 1,00 |
| Glycerin | 3,00 |
| Citronensäure | 0,40 |
| Natronlauge 45 % | 0,15 |
| EDTA-Lösung | 1,00 |
| Konservierung | 0,50 |
| Wasser | ad 100,00 |

**Beispiel 21**

| W/O-Emulsion | Gew.-% |
|---|---|
| Glyceryllanolat | 1,00 |
| Polyglyceryl-2-dipolyhydroxystearat | 4,00 |
| Mineralöl | 8,00 |
| Butylenglycoldicaprylat/Dicaproat | 12,00 |
| Isohexadecan | 6,00 |
| Dibenzoylmethan | 1,00 |
| Methylbenzylidencampher | 2,00 |
| MBTTBP | 4,00 |
| THBC | 0,20 |

(fortgesetzt)

| W/O-Emulsion | Gew.-% |
|---|---|
| Bornitrid | 2,00 |
| Titandioxid | 2,00 |
| Glycerin | 5,00 |
| Magnesiumsulfat | 0,70 |
| EDTA-Lösung | 1,00 |
| Konservierung | 0,50 |
| Wasser | ad 100,00 |

**Beispiel 22**

| W/O-Emulsion | Gew.-% |
|---|---|
| Glyceryllanolat | 1,00 |
| Polyglyceryl-2-dipolyhydroxystearat | 5,00 |
| Mineralöl | 8,00 |
| Butylenglycoldicaprylat/Dicaproat | 12,00 |
| Isohexadecan | 6,00 |
| THBC | 0,50 |
| Glycerin | 3,00 |
| Magnesiumsulfat | 0,70 |
| Konservierung | 0,50 |
| Wasser | ad 100,00 |

**Beispiel 23**

| W/O-Emulsion | Gew.-% |
|---|---|
| PEG-30-dipolyhydroxystearat | 4,00 |
| Mineralöl | 9,00 |
| Butylenglycoldicaprylat/Dicaproat | 9,00 |
| $C_{12-15}$-Alkylbenzoate | 9,00 |
| Dibenzoylmethan | 2,00 |
| Methylbenzylidencampher | 4,00 |
| MBTTBP | 4,00 |
| THBC-Sulfat | 0,50 |
| $\alpha$-Glucosylrutin | 0,50 |
| Glycerin | 3,00 |
| Magnesiumsulfat | 0,70 |
| EDTA-Lösung | 1,00 |
| Konservierung | 0,50 |
| Wasser | ad 100,00 |

**Beispiel 24**

| W/O-Emulsion | Gew.-% |
|---|---|
| PEG-30-dipolyhydroxystearat | 4,00 |
| Mineralöl | 9,00 |
| Butylenglycoldicaprylat/Dicaproat | 9,00 |
| $C_{12-15}$-Alkylbenzoate | 9,00 |
| Ectoin | 0,10 |
| T 150 | 3,00 |
| Uvasorb HEB | 2,00 |
| Octocrylene | 10,00 |
| THBC | 0,05 |
| Bornitrid | 2,00 |
| Titandioxid | 2,00 |
| Glycerin | 3,00 |
| Magnesiumsulfat , | 0,70 |
| Konservierung | 0,50 |
| Wasser | ad 100,00 |

**Beispiel 25**

| W/O-Emulsion | Gew.-% |
|---|---|
| Cetyldimethiconcopolyol | 4,00 |
| Mineralöl | 9,00 |
| Butylenglycoldicaprylat/Dicaproat | 9,00 |
| $C_{12}$-$_{15}$-Alkylbenzoate | 9,00 |
| Dibenzoylmethan | 2,00 |
| Methylbenzylidencampher | 4,00 |
| 5-Hydroxyectoin | 5,00 |
| THBC | 0,20 |
| Glycerin | 5,00 |
| Magnesiumsulfat | 0,70 |
| EDTA-Lösung | 1,00 |
| Konservierung | 0,50 |
| Wasser | ad 100,00 |

**Beispiel 26**

| W/O-Emulsion | Gew.-% |
|---|---|
| Cetyldimethiconcopolyol | 3,00 |
| Mineralöl | 9,00 |
| Butylenglycoldicaprylat/Dicaproat | 9,00 |
| $C_{12-15}$-Alkylbenzoate | 9,00 |
| Ectoin | 0,05 |
| THBC-Sulfat | 0,50 |
| Glycerin | 3,00 |

(fortgesetzt)

| W/O-Emulsion | Gew.-% |
|---|---|
| Magnesiumsulfat | 0,70 |
| EDTA-Lösung | 1,00 |
| Konservierung | 0,50 |
| Wasser | ad 100,00 |

**Beispiel 27**

| O/W-Emulsion | Gew.-% |
|---|---|
| Stearinsäure | 1,50 |
| Glycerinmonostearat | 3,00 |
| Caprylsäure/Caprinsäuretriglycerid | 10,00 |
| Dicaprylylether | 5,00 |
| Dimethicon | 2,00 |
| Hydriertes Polyisobuten | 2,00 |
| Vitamin-E-Acetat | 0,50 |
| Ectoin | 1,00 |
| Tinosorb® S | 1,00 |
| Methylbenzylidencampher | 4,00 |
| Titandioxid | 1,00 |
| THBC | 0,30 |
| Quercetin | 0,20 |
| Konservierung | 0,50 |
| Glycerin | 3,00 |
| Xanthangummi | 0,30 |
| Natronlauge 45 % | 0,50 |
| Wasser | ad 100,00 |

**Beispiel 28**

| O/W-Emulsion | Gew.-% |
|---|---|
| Sorbianstearat | 3,00 |
| Polyglyceryl-3-methylglucosedistearat | 1,50 |
| Octyldodecanol | 10,00 |
| Dicaprylylether | 5,00 |
| Mineralöl | 5,00 |
| Ricinusöl | 2,00 |
| Butylenglycoldicaprylat/caproat | 5,00 |
| Vitamin-E-Acetat | 0,50 |
| Octocrylen | 8,00 |
| Methylbenzylidencampher | 4,00 |
| Butylmethoxydibenzoylmethan | 3,00 |
| THBC-Sulfat | 0,20 |
| Rutin (wasserlöslich) | 0,50 |
| Bornitrid | 2,00 |
| 5-Hydroxyectoin | 2,00 |

(fortgesetzt)

| O/W-Emulsion | Gew.-% |
|---|---|
| Konservierung | 0,50 |
| Glycerin | 10,00 |
| Xanthangummi | 0,20 |
| Pemulen® TR1 | 0,10 |
| Phenylbenzimidazol Sulfonsäure | 2,00 |
| Natronlauge 45 % | 1,20 |
| Wasser | ad 100,00 |

**Beispiel 29**

| W/O-Emulsion | Gew.-% |
|---|---|
| Polyglyceryl-2-dipolyhydroxystearat | 5,00 |
| Dimethicon | 2,00 |
| Mineralöl | 5,00 |
| Isohexadekan | 5,00 |
| Butylenglycoldicaprylat/caproat | 10,00 |
| $C_{12-15}$-Alkylbenzoate | 7,00 |
| Dioctylbutamidotriazon | 3,00 |
| Methylbenzylidencampher | 2,00 |
| Butylmethoxydibenzoylmethan | 2,00 |
| Titandioxid | 4,00 |
| THBC | 0,20 |
| Rutin (wasserlöslich) | 0,30 |
| 5-Hydroxyectoin | 0,05 |
| Konservierung | 0,50 |
| Glycerin | 5,00 |
| $MgSO_4$ | 1,00 |
| Wasser | ad 100,00 |

**Beispiel 30**

| W/O-Emulsion | Gew.-% |
|---|---|
| PEG-30-dipolyhydroxystearat | 4,00 |
| Caprylsäure/Caprinsäuretriglycerid | 5,00 |
| Octyldodecanol | 5,00 |
| Dicaprylylether | 5,00 |
| Mineralöl | 5,00 |
| Isohexadekan | 2,00 |
| HydriertesPolyisobuten | 5,00 |
| $C_{12-14}$-Alkylbenzoate | 10,00 |
| Vitamin-E-Acetat | 0,50 |
| Aerosil® R 972 | 0,50 |
| Ectoin | 5,00 |
| THBC-Sulfat | 0,20 |
| Rutin (wasserlöslich) | 0,30 |

(fortgesetzt)

| W/O-Emulsion | Gew.-% |
|---|---|
| Konservierung | 0,50 |
| Glycerin | 10,00 |
| $MgSO_4$ | 1,00 |
| Waser | ad 100,00 |

**Beispiel 31**

| W/O-Emulsion | Gew.-% |
|---|---|
| Cetyldimethiconcopolyol | 5,00 |
| Dimethicon | 5,00 |
| Mineralöl | 2,00 |
| Isohexadekan | 2,00 |
| Butylenglycoldicaprylat/caproat | 5,00 |
| $C_{12-15}$-Alkylbenzoate | 5,00 |
| Tinosorb® S | 3,00 |
| Octocrylen | 4,00 |
| Methylbenzylidencampher | 4,00 |
| Butylmethoxydibenzoylmethan | 2,00 |
| Titandioxid | 2,00 |
| THBC | 0,30 |
| Troxerutin | 0,20 |
| Bornitrid | 4,00 |
| 5-Hydroxyectoin | 0,05 |
| Konservierung | 0,50 |
| Glycerin | 5,00 |
| NaCl | 1,00 |
| Natronlauge 45 % | 1,30 |
| Wasser | ad 100,00 |

**Beispiel 32**

| Hydrodispersion | Gew.-% |
|---|---|
| Caprylsäure/Caprinsäuretriglycerid | 10,00 |
| Octyldodecanol | 5,00 |
| Dicaprylylether | 2,00 |
| Dimethicon | 1,00 |
| Vitamin-E-Acetat | 0,50 |
| Octyltriazon | 2,00 |
| Methylbenzylidencampher | 4,00 |
| Butylmethoxydibenzoylmethan | 2,00 |
| Titandioxid | 1,00 |
| THBC | 1,00 |
| Troxerutin | 0,20 |
| Ectoin | 5,00 |
| Konservierung | 0,50 |

(fortgesetzt)

| Hydrodispersion | Gew.-% |
|---|---|
| Glycerin | 3,00 |
| Xanthangummi | 0,40 |
| Pemulen® TR 1 | 0,40 |
| Wasser | ad 100,00 |

**Beispiel 33**

| W/O-Emulsion | Gew.-% |
|---|---|
| Caprylsäure/Caprinsäuretriglycerid | 15,00 |
| Hydriertes Polyisobuten | 5,00 |
| $C_{12-15}$-Alkylbenzoate | 5,00 |
| Dioctylbutamidotriazon | 2,00 |
| Octyltriazon | 2,00 |
| Titandioxid | 4,00 |
| Aerosil® R 972 | 2,00 |
| THBC-Sulfat | 0,50 |
| Troxerutin | 0,20 |
| Bornitrid | 1,00 |
| Ectoin | 1,00 |
| Konservierung | 0,50 |
| Glycerin | 5,00 |
| NaCl | 1,00 |
| Wasser | ad 100,00 |

**Beispiel 34**

| Spray | Gew.-% |
|---|---|
| Glycerinmonostearat | 4,00 |
| Ceteareth-12 | 1,50 |
| Caprylsäure/Caprinsäuretriglyderid | 2,00 |
| Mineralöl | 5,00 |
| Octocrylen | 6,00 |
| Ectoin | 0,05 |
| THBC-Sulfat | 0,20 |
| Konservierung | 0,50 |
| Glycerin | 10,00 |
| Phenylbenzimidazol Sulfonsäure | 1,00 |
| Natronlauge 45 % | 0,40 |
| Wasser | ad 100,00 |

**Beispiel 35**

| Spray | Gew.-% |
|---|---|
| Glycerinmonostearat SE | 4,50 |
| Ceteareth-20 | 1,00 |
| Dicaprylylether | 5,00 |
| Cetylstearylisononanoat | 5,00 |
| Dimethicon | 2,00 |
| Dioctylbutamidotriazon | 1,00 |
| Tinosorb® S | 1,00 |
| Ectoin | 1,00 |
| THBC-Sulfat | 0,10 |
| Bornitrid | 1,00 |
| Konservierung | 0,50 |
| Glycerin | 5,00 |
| Wasser | ad 100,00 |

## Patentansprüche

1. Kosmetische oder pharmazeutische Formulierung, enthaltend zumindest eine Verbindung der Formel 1

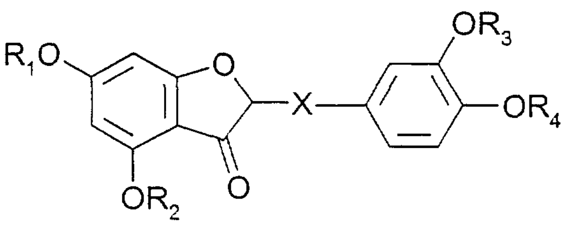

I

wobei -X- steht für eine Einfachbindung, $-CH_2-$, =CH-, -C(O)-, $=C(OR^5)-$, $-C(NR^5)-$, $-CH(NR^5R^6)-$, $-CH(OR^5)-$ und $R^1$, $R^2$, $R^3$, $R^4$, $R^5$, und $R^6$ gleich oder verschieden sein können, und unabhängig voneinander stehen für

- H
- geradkettige oder verzweigte $C_1$- bis $C_{12}$-Alkyl und/oder Alkylcarbonylgruppen,
- geradkettige oder verzweigte $C_3$- bis $C_{12}$-Alkenyl und/oder -Alkenylcarbonylgruppen,
- geradkettige oder verzweigte $C_1$- bis $C_{12}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- $C_3$- bis $C_{10}$-Cycloalkyl- und/oder Cycloalkylcarbonylgruppen und $C_3$- bis $C_{12}$-Cycloalkenyl- und/oder Cycloalkenylcarbonylgruppen, wobei die Ringe jeweils auch durch $-(CH_2)_n$-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste,

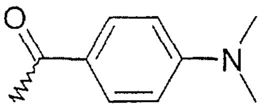

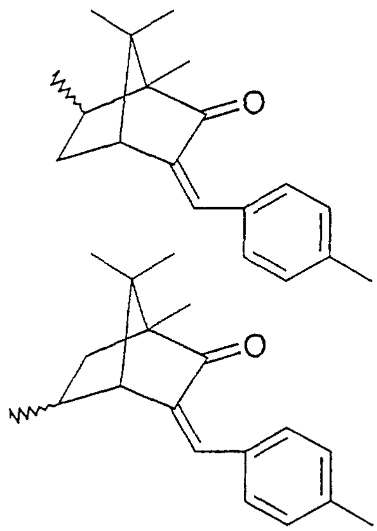

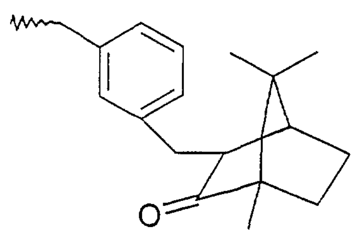

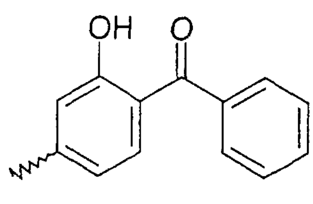

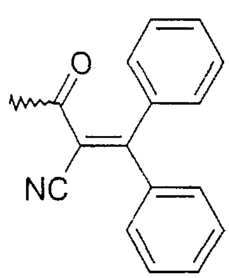

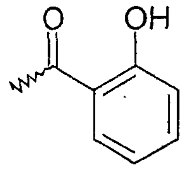

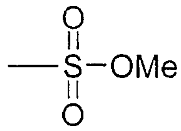

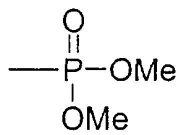

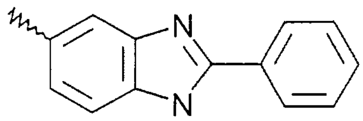

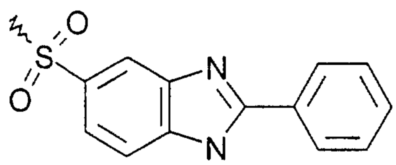

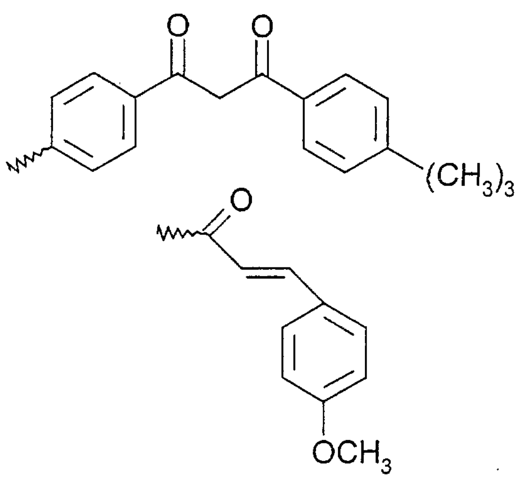

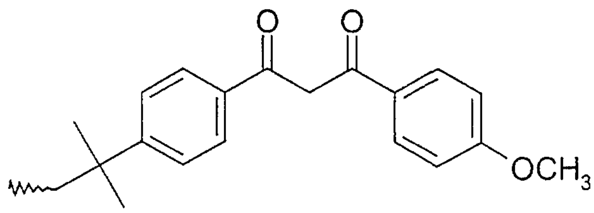

wobei Me steht für ein Proton oder ein Alkalimetallion, insbesondere ein Natrium- oder Kaliumion.

2. Kosmetische oder pharmazeutische Formulierung nach Anspruch 1, wobei -X- steht für eine Einfachbindung, $-CH_2-$, -C(O)-, $=C(OR^5)-$, $-C(NR^5)-$, $-CH(NR^5R^6)-$ oder $-CH(OR^5)-$.

3. Kosmetische oder pharmazeutische Formulierung nach Anspruch 1, wobei -X- steht für =CH- und mindestens ein Rest aus $R^1$, $R^2$, $R^3$ und $R^4$ steht für eine Gruppe, die ausgewählt ist aus der folgenden Liste

- geradkettige oder verzweigte $C_1$- bis $C_{12}$-Alkylcarbonylgruppen,
- geradkettige oder verzweigte $C_3$- bis $C_{12}$-Alkenylcarbonylgruppen,
- geradkettige oder verzweigte $C_1$- bis $C_{12}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- $C_3$- bis $C_{10}$-Cycloalkylcarbonylgruppen und $C_3$- bis $C_{12}$-Cycloalkenyl- und/oder Cycloalkenylcarbonylgruppen, wobei die Ringe jeweils auch durch $-(CH_2)_n$-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste,

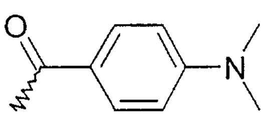

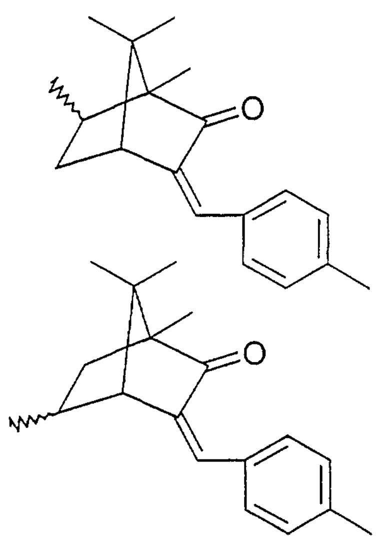

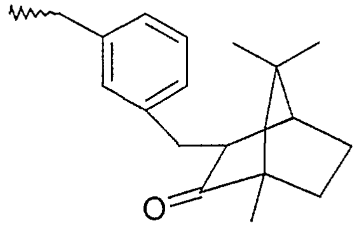

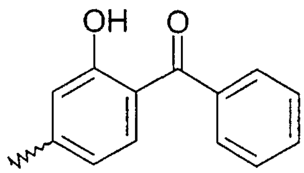

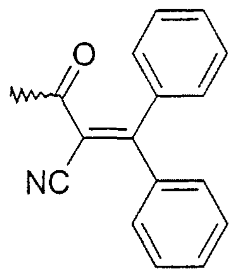

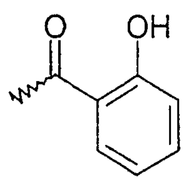

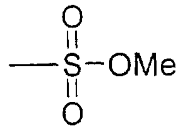

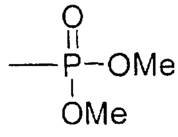

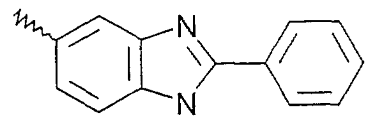

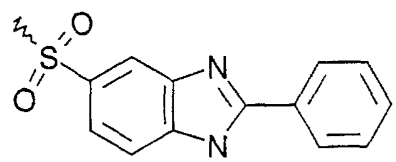

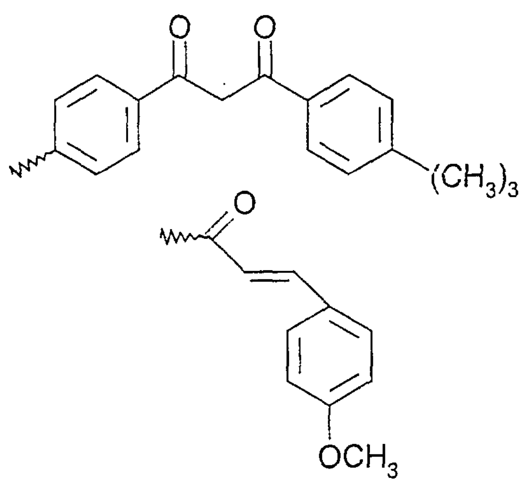

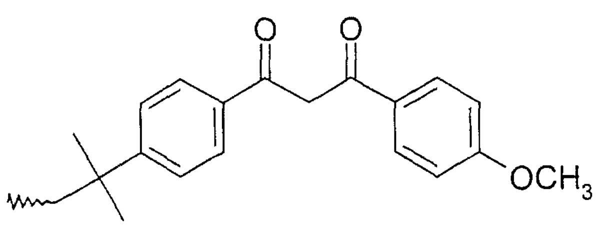

wobei Me steht für ein Proton oder ein Alkalimetallion, insbesondere ein Natrium- oder Kaliumion.

4. Kosmetische oder pharmazeutische Formulierung nach mindestens einem der vorhergehenden Ansprüche, wobei es sich bei der Verbindung der Formel I um 4,6,3',4'-Tetrahydroxybenzylcoumaranon-3 (X = $-CH_2-$ und $R^1 = R^2 = R^3 = R^4 = H$) oder ein Derivat davon, vorzugsweise das Trisulfat (X = $-CH_2-$; $R^1 = R^3 = R^4 = SO_3Me$, $R^2 = H$) handelt.

5. Kosmetische oder pharmazeutische Formulierung nach mindestens einem der vorhergehenden Ansprüche, wobei die Formulierung einen oder mehrere UV-Filter enthält, die vorzugsweise ausgewählt sind aus der Gruppe, die 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

6. Kosmetische oder pharmazeutische Formulierung nach mindestens einem der vorhergehenden Ansprüche zum Schutz von Körperzellen gegen oxidativen Stress, insbesondere zur Verringerung der Hautalterung, die vorzugsweise ein oder mehrere Antioxidantien enthält.

7. Kosmetische oder pharmazeutische Formulierung nach mindestens einem der vorausgehenden Ansprüche, **dadurch gekennzeichnet, dass** in der Formulierung weitere hautschonende oder hautpflegende Wirkstoffe enthalten sind, wobei vorzugsweise Pyrimidincarbonsäuren gemäß Formel II,

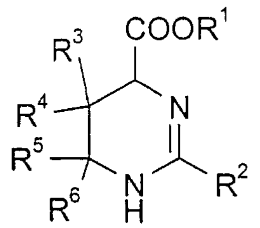

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind, und/oder Aryloxime, insbesondere 2-Hydroxy-5-methyllaurophenonoxim enthalten sind.

8. Kosmetische oder pharmazeutische Formulierung nach Anspruch 7, **dadurch gekennzeichnet, dass** Pyrimidincarbonsäuren gemäß Formel II enthalten sind, bei denen $R^2$ eine Methyl- oder eine Ethylgruppe ist und $R^1$ bzw. $R^5$ und $R^6$ H sind, wobei es sich vorzugsweise um mindestens eine der Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidincarbonsäure) handelt.

9. Kosmetische oder pharmazeutische Formulierung nach mindestens einem der vorausgehenden Ansprüche, **dadurch gekennzeichnet, dass** es sich bei der Formulierung um eine Emulsion handelt, die O/W- oder W/O-Emulgatoren enthält, wobei bevorzugte W/O-Emulgatoren ausgewählt sind aus Glycerylmonostearat, Glycerylmonoisostearat, Glyrerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

10. Kosmetische oder pharmazeutische Formulierung nach mindestens einem der vorausgehenden Ansprüche, **dadurch gekennzeichnet, dass** die Formulierung mindestens ein hydrophiles Tensid, bevorzugt gewählt aus der Gruppe der Alkylglucoside, der Acyllactylate, der Betaine sowie der Cocoamphoacetate, enthält.

11. Nahrungsmittel, umfassend zumindest eine Verbindung der Formel I, wobei die Variablen die in Anspruch 1 angegebene Bedeutung aufweisen.

12. Nahrungsmittel nach Anspruch 11, weiter umfassend Vitamin C und/oder ein Vitamin C-Derivat.

13. Verbindungen der Formel I, wobei die Variablen die in Anspruch 2 angegebene Bedeutung aufweisen, als Arzneimittel.

14. Verbindungen der Formel I, wobei die Variablen die in Anspruch 3 angegebene Bedeutung aufweisen, als Arzneimittel.

15. Verwendung einer Verbindung der Formel I, wobei die Variablen die in Anspruch 2 angegebene Bedeutung aufweisen, zur Herstellung eines Arzneimittels gegen oxidativen Stress, insbesondere zur Verminderung der Hautalterung.

16. Verwendung einer Verbindung der Formel I, wobei die Variablen die in Anspruch 2 angegebene Bedeutung aufweisen, zur Herstellung eines Arzneimittels zur Behandlung von Entzündungen oder allergischen Reaktionen.

17. Verbindung der Formel I

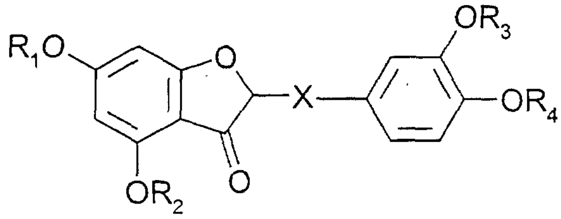

I

wobei die Variablen die in Anspruch 1 angegebene Bedeutung aufweisen, **dadurch kennzeichnet, dass** mindestens ein Rest aus $R^1$, $R^2$, $R^3$ und $R^4$ nicht H ist.

**18.** Verbindung nach Anspruch 17, **dadurch gekennzeichnet, dass** mindestens ein Rest aus $R^1$, $R^2$, $R^3$ und $R^4$ eine Gruppe $-SO_3Me$ oder $-PO_3Me_2$ ist, wobei es sich insbesondere bevorzugt um eine Verbindung der Formel III

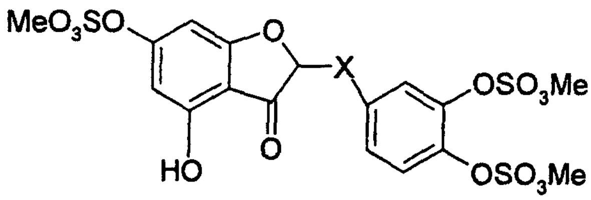

III

die rein oder in Mischung mit den entsprechenden Mono- und Disulfaten vorliegen kann, handelt, wobei das Trisulfat von 4,6,3',4'-Tetrahydroxybenzylcoumaranon-3 insbesondere bevorzugt ist.

**19.** Verbindung nach mindestens einem der Ansprüche 17 oder 18, **dadurch gekennzeichnet, dass** wobei -X- steht für eine Einfachbindung, $-CH_2-$, -C(O)-, $=C(OR^5)-$, $-C(NR^5)-$, $-CH(NR^5R^6)-$ oder $-CH(OR^5)-$.

**20.** Verwendung einer Verbindung der Formel I, wobei -X- steht für eine Einfachbindung, $-CH_2-$, -C(O)-, $=C(OR^5)-$, $-C(NR^5)-$, $-CH(NR^5R^6)-$oder $-CH(OR^5)-$ und die übrigen Variablen die in Anspruch 1 angegebene Bedeutung aufweisen, als Antioxidationsmittel, insbesondere für kosmetische Formulierungen oder Nahrungsmittel.

**21.** Verwendung von Verbindungen der Formel I, wobei -X- steht für eine Einfachbindung, $-CH_2-$, -C(O)-, $=C(OR^5)-$, $-C(NR^5)-$, $-CH(NR^5R^6)-$oder $-CH(OR^5)-$ und die übrigen Variablen die in Anspruch 1 angegebene Bedeutung aufweisen, zur Stabilisierung von UV-Filtern, insbesondere Dibenzoylmethan und Derivaten des Dibenzoylmethans.

**22.** Verwendung von Verbindungen nach Formel I mit Bedeutung der Reste entsprechend Anspruch 1 zur Herstellung einer Zubereitung geeignet zur Immunprotektion.

**23.** Verwendung von Verbindungen nach Formel I mit Bedeutung der Reste entsprechend Anspruch 1 zur Herstellung einer Zubereitung geeignet zum Schutz der DNA und RNA.

**24.** Verwendung von Verbindungen nach Formel I mit Bedeutung der Reste entsprechend Anspruch 1 zur Herstellung einer Zubereitung geeignet zum Schutz von Zellen, insbesondere von Langerhans-Zellen.

## Claims

**1.** Cosmetic or pharmaceutical formulation comprising at least one compound of the formula I

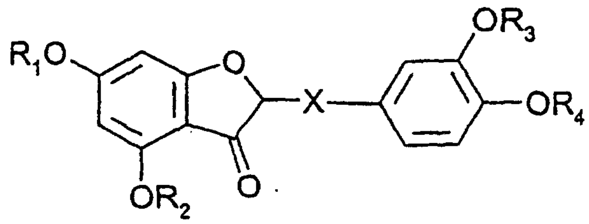

I

in which -X- stands for a single bond, $-CH_2-$, =CH-, -C(O)-, $=C(OR^5)-$, $-C(NR^5)-$, $-CH(NR^5R^6)-$ or $-CH(OR^5)-$, and $R^1$, $R^2$, $R^3$, $R^4$, $R^5$ and $R^6$ may be identical or different and, independently of one another, stand for

- H
- straight-chain or branched $C_1$- to $C_{12}$-alkyl and/or alkylcarbonyl groups,
- straight-chain or branched $C_3$- to $C_{12}$-alkenyl and/or -alkenylcarbonyl groups,
- straight-chain or branched $C_1$- to $C_{12}$-hydroxyalkyl groups, in which the hydroxyl group may be bonded to a primary or secondary carbon atom of the chain, and furthermore the alkyl chain may also be interrupted by oxygen,
- $C_3$- to $C_{10}$-cycloalkyl and/or cycloalkylcarbonyl groups and $C_3$- to $C_{12}$-cycloalkenyl and/or cycloalkenylcarbonyl groups, in which each of the rings may also be bridged by $-(CH_2)_n-$ groups, where n = 1 to 3,
- aryl and/or arylcarbonyl groups,
- heteroaryl and/or heteroarylcarbonyl groups, where these groups may be substituted by alkyl, hydroxyl, alkoxy, amino, mono- and dialkylamino, sulfonic acid, carboxyl and/or halogen groups,
- mono- and/or oligoglycosyl radicals,

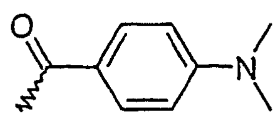

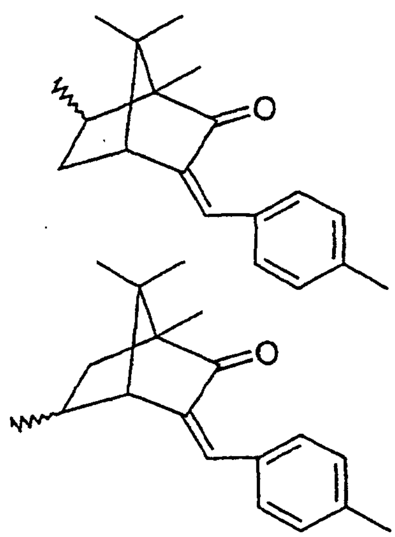

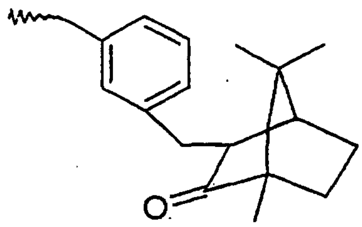

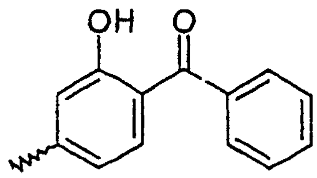

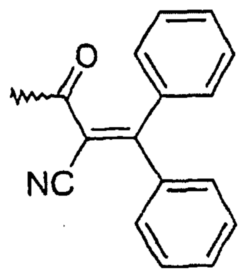

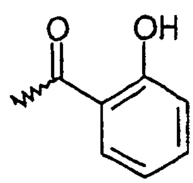

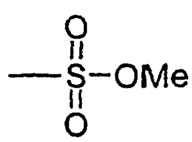

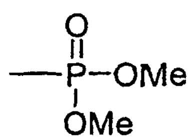

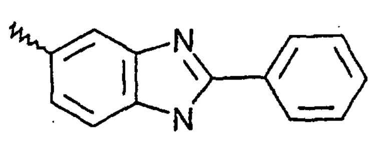

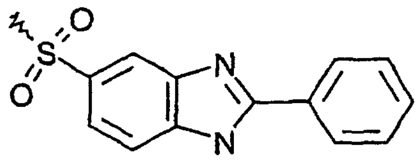

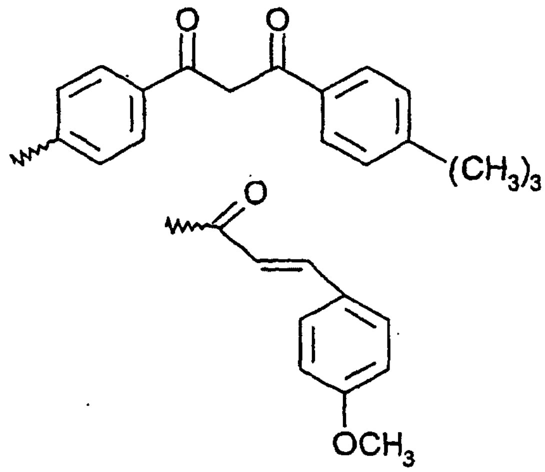

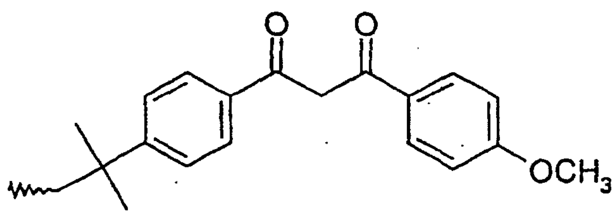

in which Me stands for a proton or an alkali metal ion, in particular a sodium or potassium ion.

**2.** Cosmetic or pharmaceutical formulation according to Claim 1, in which -X- stands for a single bond, $-CH_2-$, -C(O)-, $=C(OR^5)-$, $-C(NR^5)-$, $-CH(NR^5R^6)-$ or $-CH(OR^5)-$.

**3.** Cosmetic or pharmaceutical formulation according to Claim 1, in which -X- stands for =CH-, and at least one radical from $R^1$, $R^2$, $R^3$ and $R^4$ stands for a group selected from the following list

- straight-chain or branched $C_1$- to $C_{12}$-alkylcarbonyl groups,
- straight-chain or branched $C_3$- to $C_{12}$-alkenylcarbonyl groups,
- straight-chain or branched $C_1$- to $C_{12}$-hydroxyalkyl groups, in which the hydroxyl group may be bonded to a primary or secondary carbon atom of the chain, and furthermore the alkyl chain may also be interrupted by oxygen,
- $C_3$- to $C_{10}$-cycloalkylcarbonyl groups and $C_3$- to $C_{12}$-cycloalkenyl and/or cycloalkenylcarbonyl groups, in which each of the rings may also be bridged by $-(CH_2)_n-$ groups, where n = 1 to 3,
- aryl and/or arylcarbonyl groups,
- heteroaryl and/or heteroarylcarbonyl groups, where these groups may be substituted by alkyl, hydroxyl, alkoxy, amino, mono- and dialkylamino, sulfonic acid, carboxyl and/or halogen groups,
- mono- and/or oligoglycosyl radicals,

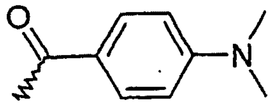

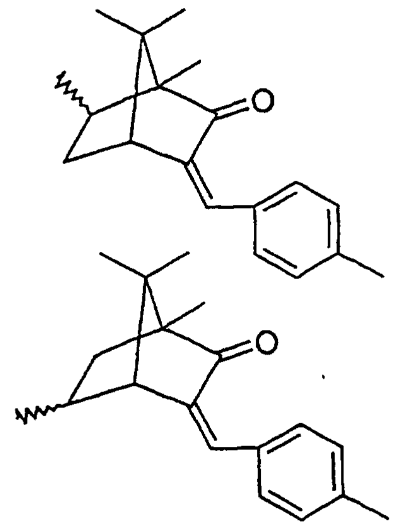

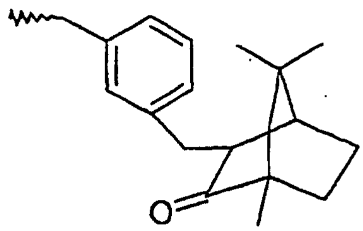

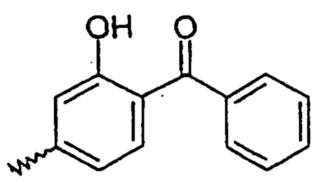

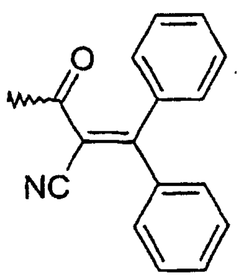

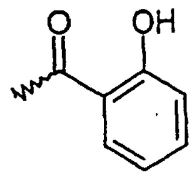

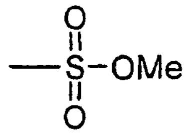

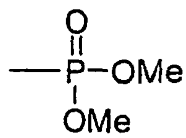

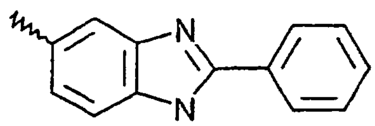

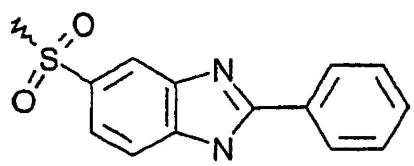

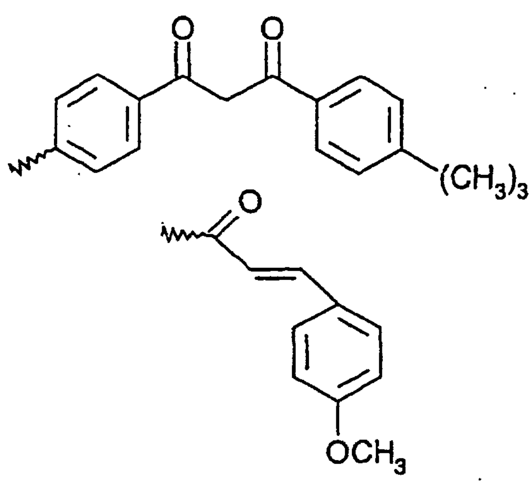

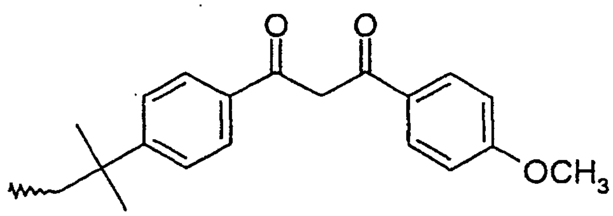

in which Me stands for a proton or an alkali metal ion, in particular a sodium or potassium ion.

4. Cosmetic or pharmaceutical formulation according to at least one of the preceding claims, where the compound of the formula I is 4,6,3',4'-tetrahydroxybenzylcoumaran-3-one (X = $-CH_2-$ and $R^1 = R^2 = R^3 = R^4 = H$) or a derivative thereof, preferably the trisulfate (X = $-CH_2-$; $R^1 = R^3 = R^4 = SO_3Me$, $R^2 = H$).

5. Cosmetic or pharmaceutical formulation according to at least one of the preceding claims, where the formulation comprises one or more UV filters, which are preferably selected from the group which comprises 3-(4'-methylbenzylidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and the potassium, sodium and triethanolamine salts thereof.

6. Cosmetic or pharmaceutical formulation according to at least one of the preceding claims for protecting body cells against oxidative stress, in particular for reducing skin ageing, which preferably comprises one or more antioxidants.

7. Cosmetic or pharmaceutical formulation according to at least one of the preceding claims, **characterised in that** the formulation comprises further skin-protecting or skin-care active ingredients, preferably pyrimidinecarboxylic acids of the formula II

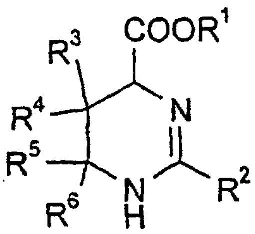

II

in which $R^1$ is a radical H or C1-8-alkyl, $R^2$ is a radical H or C1-4-alkyl, and $R^3$, $R^4$, $R^5$ and $R^6$ are each, independently of one another, a radical from the group H, OH, $NH_2$ and C1-4-alkyl, and/or aryl oximes, in particular 2-hydroxy-5-methyllaurophenone oxime.

8. Cosmetic or pharmaceutical formulation according to Claim 7, **characterised in that** pyrimidinecarboxylic acids of the formula II are present in which $R^2$ is a methyl or ethyl group, and $R^1$ or $R^5$ and $R^6$ are H, where at least one of the pyrimidinecarboxylic acids is preferably ectoine ((S)-1,4,5,6-tetrahydro-2-methyl-4-pyrimidinecarboxylic acid) or hydroxyectoine ((S,S)-1,4,5,6-tetrahydro-5-hydroxy-2-methyl-4-pyrimidinecarboxylic acid).

9. Cosmetic or pharmaceutical formulation according to at least one of the preceding claims, **characterised in that** the formulation is an emulsion which comprises O/W or W/O emulsifiers, with preferred W/O emulsifiers being selected from glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate, glyceryl monocaprylate.

10. Cosmetic or pharmaceutical formulation according to at least one of the preceding claims, **characterised in that**

the formulation comprises at least one hydrophilic surfactant, preferably selected from the group of the alkyl glucosides, acyl lactylates, betaines and coconut amphoacetates.

**11.** Food comprising at least one compound of the formula I where the variables have the meaning indicated in Claim 1.

**12.** Food according to Claim 11, further comprising vitamin C and/or a vitamin C derivative.

**13.** Compounds of the formula I where the variables have the meaning indicated in Claim 2, as medicaments.

**14.** Compounds of the formula I where the variables have the meaning indicated in Claim 3, as medicaments.

**15.** Use of a compound of the formula I where the variables have the meaning indicated in Claim 2, for the preparation of a medicament against oxidative stress, in particular for reducing skin ageing.

**16.** Use of a compound of the formula I where the variables have the meaning indicated in Claim 2, for the preparation of a medicament for the treatment of inflammation or allergic reactions.

**17.** Compound of the formula I

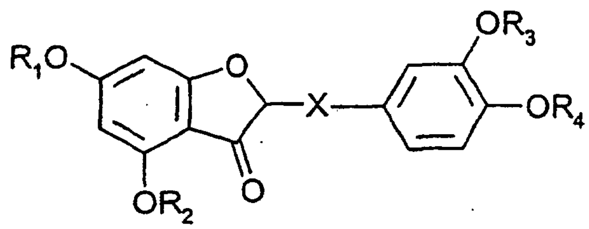

I

where the variables have the meaning indicated in Claim 1, **characterised in that** at least one radical from $R^1$, $R^2$, $R^3$ and $R^4$ is not H.

**18.** Compound according to Claim 17, **characterised in that** at least one radical from $R^1$, $R^2$, $R^3$ and $R^4$ is an $-SO_3Me$ or $-PO_3Me_2$ group, which is particularly preferably a compound of the formula III

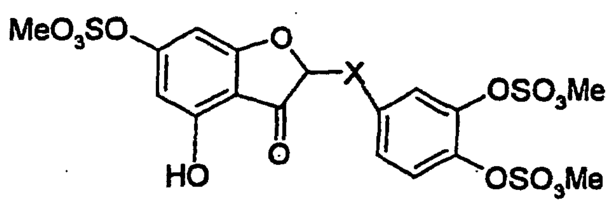

III

which may be pure or in a mixture with the corresponding mono- and disulfates, where the trisulfate of 4,6,3',4'-tetrahydroxybenzylcoumaran-3-one is particularly preferred.

**19.** Compound according to at least one of Claims 17 and 18, **characterised in that** -X- stands for a single bond, $-CH_2-$, -C(O)-, $=C(OR^5)-$, $-C(NR^5)-$, $-CH(NR^5R^6)-$ or $-CH(OR^5)-$.

**20.** Use of a compound of the formula I in which -X- stands for a single bond, $-CH_2-$, -C(O)-, $=C(OR^5)-$, $-C(NR^5)-$, $-CH(NR^5R^6)-$ or $-CH(OR^5)-$, and the other variables have the meaning indicated in Claim 1, as antioxidant, in particular for cosmetic formulations or foods.

**21.** Use of compounds of the formula I in which -X- stands for a single bond, $-CH_2-$, -C(O)-, $=C(OR^5)-$, $-C(NR^5)-$, $-CH(NR^5R^6)-$ or $-CH(OR^5)-$, and the other variables have the meaning indicated in Claim 1, for the stabilisation of UV filters, in particular dibenzoylmethane and derivatives of dibenzoylmethane.

22. Use of compounds of the formula I with the meaning of the radicals corresponding to Claim 1 for the preparation of a preparation suitable for immunoprotection.

23. Use of compounds of the formula I with the meaning of the radicals corresponding to Claim 1 for the preparation of a preparation suitable for the protection of DNA and RNA.

24. Use of compounds of the formula I with the meaning of the radicals corresponding to Claim 1 for the preparation of a preparation suitable for the protection of cells, in particular of Langerhans cells.

## Revendications

1. Formulation cosmétique ou pharmaceutique comprenant au moins un composé de la formule I

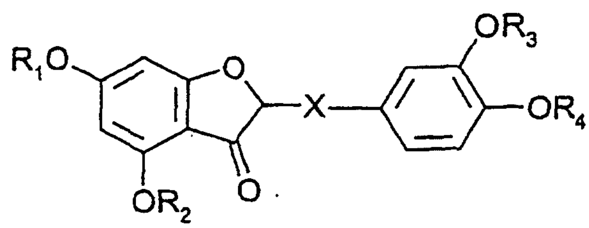

I

dans laquelle -X- représente une liaison simple, $-CH_2-$, =CH-, -C(O)-, $=C(OR^5)-$, $-C(NR^5)-$, $-CH(NR^5R^6)-$ ou $-CH(OR^5)-$, et $R^1$, $R^2$, $R^3$, $R^4$, $R^5$ et $R^6$ peuvent être identiques ou différents et, indépendamment les uns des autres, peuvent représenter

- H
- des groupes $C_1$- à $C_{12}$-alkyle et/ou alkylcarbonyle à chaîne droite ou ramifiée,
- des groupes $C_3$- à $C_{12}$-alkényle et/ou -alkénylcarbonyle à chaîne droite ou ramifiée,
- des groupes $C_1$- à $C_{12}$-hydroxyalkyle à chaîne droite ou ramifiée, dans lesquels le groupe hydroxyle peut être lié à un atome de carbone primaire ou secondaire de la chaîne, et en outre la chaîne alkyle peut également être interrompue par oxygène,
- des groupes $C_3$- à $C_{10}$-cycloalkyle et/ou cycloalkylcarbonyle et des groupes $C_3$- à $C_{12}$-cycloalkényle et/ou cycloalkénylcarbonyle, dans lesquels chacun des cycles peut également être ponté par des groupes $-(CH_2)_n-$, où n = 1 à 3,
- des groupes aryle et/ou arylcarbonyle,
- des groupes hétéroaryle et/ou hétéroarylcarbonyle, dans lesquels ces groupes peuvent être substitués par des groupes alkyle, hydroxyle, alkoxy, amino, mono- et dialkylamino, acide sulfonique, carboxyle et/ou halogène,
- des radicaux mono- et/ou oligoglycosyle,

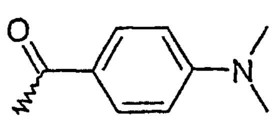

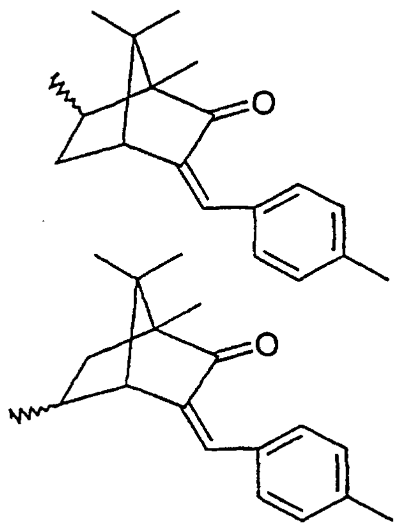

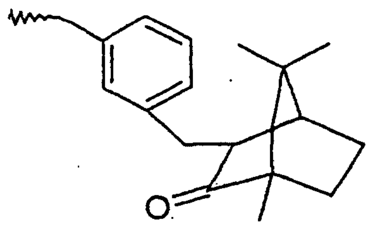

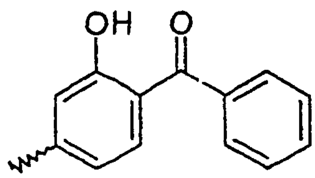

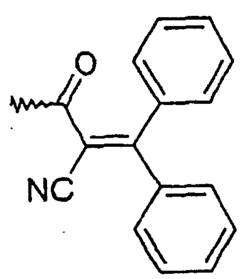

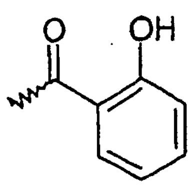

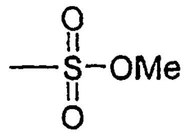

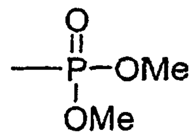

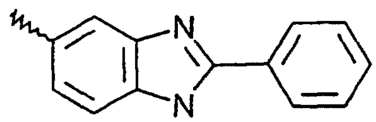

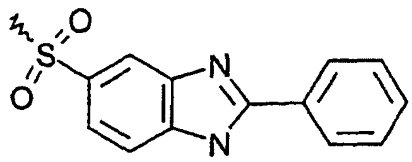

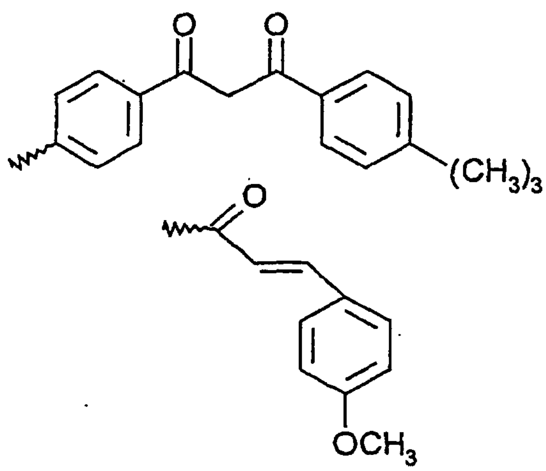

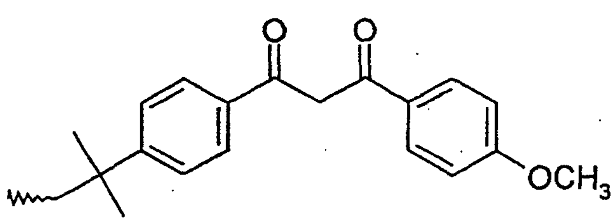

dans lesquels Me représente un proton ou un ion de métal alcalin, en particulier un ion sodium ou potassium.

**2.** Formulation cosmétique ou pharmaceutique selon la revendication 1, dans laquelle -X- représente une liaison simple, $-CH_2-$, -C(O)-, $=C(OR^5)-$, $-C(NR^5)-$, $-CH(NR^5R^6)-$ ou $-CH(OR^5)-$.

**3.** Formulation cosmétique ou pharmaceutique selon la revendication 1, dans laquelle -X- représente =CH-, et au moins un radical parmi $R^1$, $R^2$, $R^3$ et $R^4$ représente un groupe choisi parmi la liste qui suit :

- des groupes $C_1$- à $C_{12}$-alkylcarbonyle à chaîne droite ou ramifiée,
- des groupes $C_3$- à $C_{12}$-alkénylcarbonyle à chaîne droite ou ramifiée,
- des groupes $C_1$- à $C_{12}$-hydroxyalkyle à chaîne droite ou ramifiée, dans lesquels le groupe hydroxyle peut être lié à un atome de carbone primaire ou secondaire de la chaîne, et en outre la chaîne alkyle peut également être interrompue par oxygène,
- des groupes $C_3$- à $C_{10}$-cycloalkylcarbonyle et des groupes $C_3$- à $C_{12}$-cycloalkenyle et/ou cycloalkénylcarbonyle, dans lesquels chacun des cycles peut également être ponté par des groupes $-(CH_2)_n-$, où n = 1 à 3,
- des groupes aryle et/ou arylcarbonyle,
- des groupes hétéroaryle et/ou hétéroarylcarbonyle, dans lesquels ces groupes peuvent être substitués par des groupes alkyle, hydroxyle, alkoxy, amino, mono- et dialkylamino, acide sulfonique, carboxyle et/ou halogène,
- des radicaux mono- et/ou oligoglycosyle,

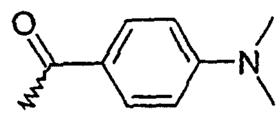

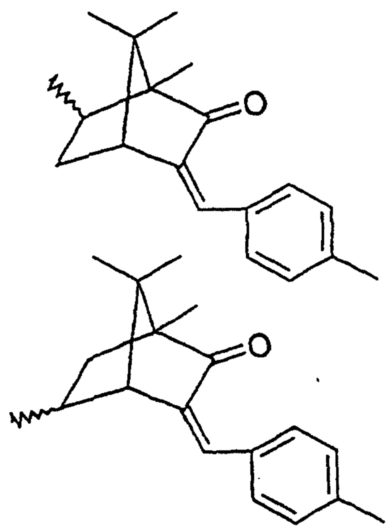

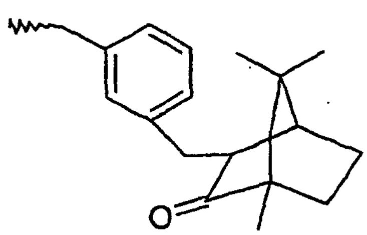

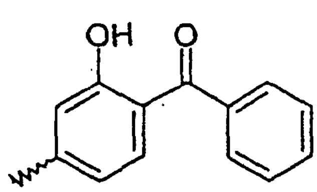

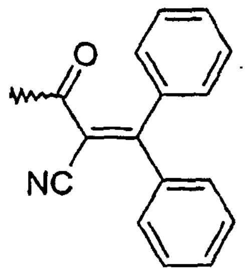

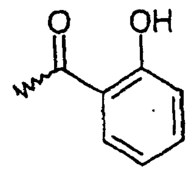

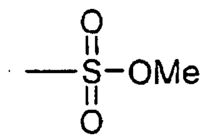

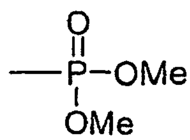

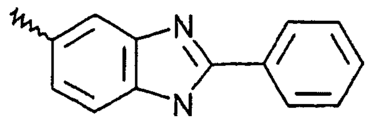

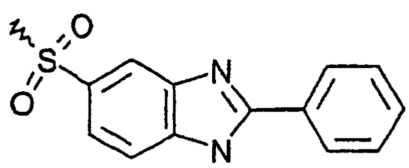

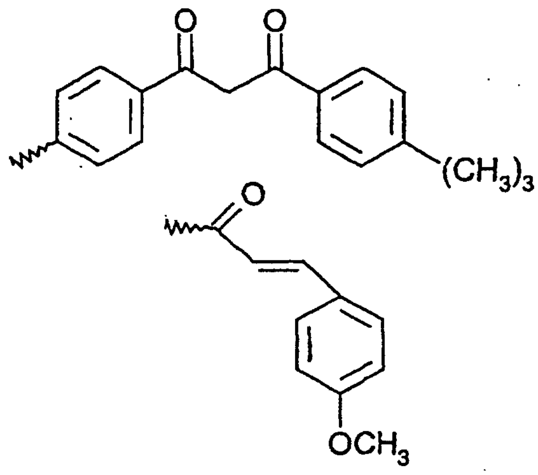

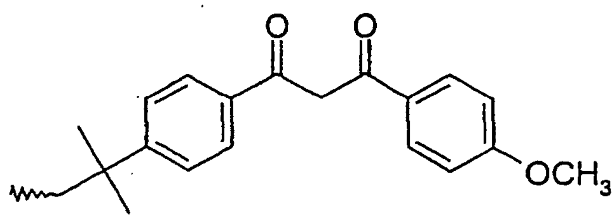

dans lesquels Me représente un proton ou un ion de métal alcalin, en particulier un ion sodium ou potassium.

4. Formulation cosmétique ou pharmaceutique selon au moins l'une des revendications précédentes, dans lequel le composé de la formule I est 4,6,3',4'-tétrahydroxybenzylcoumarine-3-one (X = $-CH_2-$ et $R^1 = R^2 = R^3 = R^4 = H$) ou un dérivé afférent, de préférence le trisulfate (X = $-CH_2-$; $R^1 = R^3 = R^4 = SO_3Me$, $R^2 = H$).

5. Formulation cosmétique ou pharmaceutique selon au moins l'une des revendications précédentes, dans lequel la formulation comprend un ou plusieurs filtres UV, qui sont de préférence choisis parmi le groupe comprenant 3-(4'-méthylbenzylidène)-dl-camphre, 1-(4-tert-butylphényl)-3-(4-méthoxyphényl)propane-1,3-dione, 4-isopropyldibenzoylméthane, 2-hydroxy-4-méthoxybenzophénone, octyle méthoxycinnamate, 3,3,5-triméthylcyclohexyle salicylate, 2-éthylhexyle 4-(diméthylamino)benzoate, 2-éthylhexyle 2-cyano-3,3-diphénylacrylate, acide 2-phénylbenzimidazole-5-sulfonique et les sels de potassium, sodium et triéthanolamine afférents.

6. Formulation cosmétique ou pharmaceutique selon au moins l'une des revendications précédentes pour protéger les cellules corporelles contre un stress oxydant, en particulier pour réduire le vieillissement de la peau, qui comprend de préférence un ou plusieurs antioxydants.

7. Formulation cosmétique ou pharmaceutique selon au moins l'une des revendications précédentes, **caractérisée en ce que** la formulation comprend en outre des ingrédients actifs de protection de peau ou de soin de peau, de préférence des acides pyrimidinecarboxyliques de la formule II

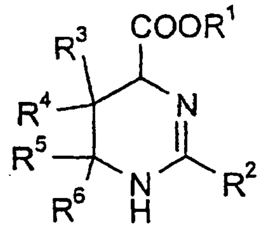

II

dans laquelle $R^1$ est un radical H ou C1-8-alkyle, $R^2$ est un radical H ou C1-4-alkyle, et $R^3$, $R^4$, $R^5$ et $R^6$ sont chacun, indépendamment l'un de l'autre, un radical pris parmi le groupe des oxymes H, OH, $NH_2$ et C1-4-alkyle et/ou aryle, en particulier l'oxyme 2-hydroxy-5-méthyllaurophénone.

**8.** Formulation cosmétique ou pharmaceutique selon la revendication 7, **caractérisée en ce que** des acides pyrimidinecarboxyliques de la formule II sont présents, dans lesquels $R^2$ est un groupe méthyle ou éthyle, et $R^1$ ou $R^5$ et $R^6$ sont H, dans laquelle au moins l'un des acides pyrimidinecarboxyliques est de préférence ectoïne (acide (S)-1,4,5,6-tétrahydro-2-méthyl-4-pyrimidinecarboxylique) ou hydroxyectoïne (acide (S,S)-1,4,5,6-tétrahydro-5-hydroxy-2-méthyl-4-pyrimidinecarboxylique).

**9.** Formulation cosmétique ou pharmaceutique selon au moins l'une des revendications précédentes, **caractérisée en ce que** la formulation est une émulsion qui comprend des émulsifiants O/W ou W/O, des émulsifiants W/O préférés étant choisis parmi monostéarate de glycéryle, monoisostéarate de glycéryle, monomyristate de glycéryle, monooléate de glycéryle, monostéarate de diglycéryle, monoisostéarate de diglycéryle, monostéarate de propylène glycol, monoisostéarate de propylène glycol, monocaprylate de propylène glycol, monolaurate de propylène glycol, monoisostéarate de sorbitan, monolaurate de sorbitan, monocaprylate de sorbitan, monoisooléate de sorbitan, distéarate de sucrose, alcool de cétyle, alcool de stéaryle, alcool d'arachidyle, alcool de béhényle, alcool d'isobéhényle, alcool de sélachyle, alcool de chimylel, éther de polyéthylène glycol (2) stéaryle (stéaréth-2), monolaurate de glycéryle, monocaprinate de glycéryle, monocaprylate de glycéryle.

**10.** Formulation cosmétique ou pharmaceutique selon au moins l'une des revendications précédentes, **caractérisée en ce que** la formulation comprend au moins un agent tensioactif hydrophile, de préférence choisi parmi le groupe des glucosides d'alkyle, lactylates d'acyle, bétaïnes et amphoacétates de noix de coco.

**11.** Aliment comprenant au moins un composé de la formule I dans laquelle les variables ont la signification indiquée dans la revendication 1.

**12.** Aliment selon la revendication 11, comprenant en outre de la vitamine C et/ou un dérivé de vitamine C.

**13.** Composés de la formule I dans lesquels les variables ont les significations indiquées dans la revendication 2, en tant que médicaments.

**14.** Composés de la formule I dans lesquels les variables ont les significations indiquées dans la revendication 3, en tant que médicaments.

**15.** Utilisation d'un composés de la formule I dans lequel les variables ont les significations indiquées dans la revendication 2, pour la préparation d'un médicament contre le stress oxydant, en particulier pour réduire le vieillissement de la peau.

**16.** Utilisation d'un composés de la formule I dans lequel les variables ont les significations indiquées dans la revendication 2, pour la préparation d'un médicament pour le traitement de réactions inflammatoires ou allergiques.

**17.** Composé de la formule I

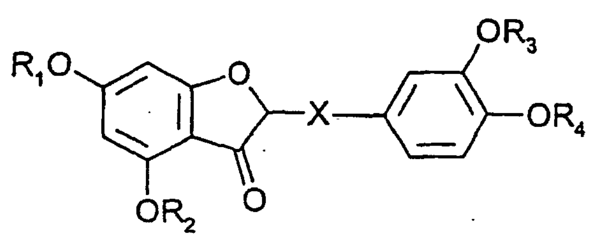

I

dans laquelle les variables ont la signification indiquée dans la revendication 1, **caractérisé en ce qu'**au moins un radical pris parmi $R^1$, $R^2$, $R^3$ et $R^4$ n'est pas H.

**18.** Composé selon la revendication 17, **caractérisé en ce qu'**au moins un radical pris parmi $R^1$, $R^2$, $R^3$ et $R^4$ est un groupe $-SO_3Me$ ou $-PO_3Me_2$, lequel est particulièrement de préférence un composé de la formule III

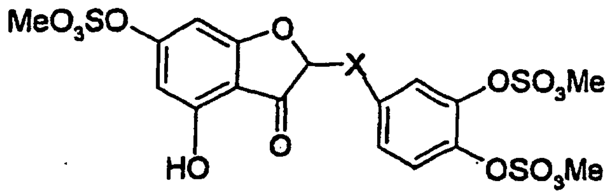

III

qui peut être pur ou dans un mélange avec les mono- et disulfates correspondants, dans lequel le trisulfate de 4,6,3',4'-tétrahydroxybenzylcoumaran-3-one est tout particulièrement préféré.

**19.** Composé selon au moins l'une des revendications 17 et 18, **caractérisé en ce que** -X- représente une liaison simple, $-CH_2-$, -C(O)-, $=C(OR^5)-$, $-C(NR^5)-$, $-CH(NR^5R^6)-$ ou $-CH(OR^5)-$.

**20.** Utilisation d'un composé de la formule I dans laquelle -X- représente une liaison simple, $-CH_2-$, -C(O)-, $=C(OR^5)-$, $-C(NR^5)-$, $-CH(NR^5R^6)-$ou $-CH(OR^5)-$, et les autre variables ont la signification indiquée dans la revendication 1, en tant qu'antioxydant, en particulier pour des formulations cosmétiques ou des aliments.

**21.** Utilisation de composés de la formule I dans laquelle -X- représente une liaison simple, $-CH_2-$, -C(O)-, $=C(OR^5)-$, $-C(NR^5)-$, $-CH(NR^5R^6)-$ou $-CH(OR^5)-$,et les autre variables ont la signification indiquée dans la revendication 1, pour la stabilisation de filtres UV, en particulier dibenzoylméthane et des dérivés de dibenzoylméthane.

**22.** Utilisation de composés de la formule I avec la signification des radicaux correspondant à la revendication 1 pour la préparation d'une préparation convenant pour une immunoprotection.

**23.** Utilisation de composés de la formule I avec la signification des radicaux correspondant à la revendication 1 pour la préparation d'une préparation convenant pour la protection d'ADN et d'ARN.

**24.** Utilisation de composés de la formule I avec la signification des radicaux correspondant à la revendication 1 pour la préparation d'une préparation convenant pour la protection de cellules, en particulier de cellules de Langerhans.